(19) Europäisches Patentamt
European Patent Office
Office européen des brevets

(11) **EP 3 646 835 B1**

(12) **EUROPEAN PATENT SPECIFICATION**

(45) Date of publication and mention
of the grant of the patent:
**03.05.2023   Bulletin 2023/18**

(21) Application number: **18824215.0**

(22) Date of filing: **28.06.2018**

(51) International Patent Classification (IPC):
**A61F 13/511** *(2006.01)*    **A61F 13/512** *(2006.01)*
**A61F 13/514** *(2006.01)*    **D04H 1/495** *(2012.01)*
**A61F 13/15** *(2006.01)*    **D04H 1/02** *(2006.01)*

(52) Cooperative Patent Classification (CPC):
**A61F 13/15203; A61F 13/511; A61F 13/512;**
**A61F 13/514; A61F 13/51401; D04H 1/02;**
**D04H 1/495;** A61F 2013/15406; A61F 2013/15422;
A61F 2013/51019

(86) International application number:
**PCT/JP2018/024642**

(87) International publication number:
**WO 2019/004369 (03.01.2019 Gazette 2019/01)**

(54) **SHEET FOR ABSORBENT ARTICLE, AND ABSORBENT ARTICLE**

BLATT FÜR SAUGFÄHIGEN ARTIKEL UND SAUGFÄHIGER ARTIKEL

FEUILLE POUR ARTICLE ABSORBANT, ET ARTICLE ABSORBANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:   **30.06.2017   JP 2017129525**

(43) Date of publication of application:
**06.05.2020   Bulletin 2020/19**

(73) Proprietors:
• **Daiwabo Holdings Co., Ltd.**
**Osaka-shi, Osaka 541-0056 (JP)**
• **Daiwabo Polytec Co., Ltd.**
**Osaka-shi, Osaka 541-0056 (JP)**

(72) Inventor: **MAKIHARA, Hiroko**
**Kako-gun, Hyogo 675-0163 (JP)**

(74) Representative: **Eisenführ Speiser**
**Patentanwälte Rechtsanwälte PartGmbB**
**Postfach 10 60 78**
**28060 Bremen (DE)**

(56) References cited:
WO-A1-2016/108285        JP-A- H1 193 055
JP-A- H03 137 258        JP-A- H10 280 260
JP-A- 2010 106 430        JP-A- 2011 137 279
JP-A- 2015 180 789        JP-A- 2017 113 040

**Description**

Technical Field

The present disclosure relates to a sheet for an absorbent article and an absorbent article using the same.

Background Art

Nonwoven fabrics with various structures have been proposed as sheets constituting absorbent articles, such as sanitary napkins, disposable diapers, and incontinence pads, that is, sheets for absorbent articles. For example, Patent Document 1 mentions that a surface sheet for an absorbent article is formed by a nonwoven fabric that contains 50% by mass or more of non-heat-fusible hydrophilic fibers and is formed to have a concavo-convex structure in which streaky convex portions and concave portions both extending in the longitudinal direction are alternately arranged in the width direction. In the nonwoven fabric, the density of the fibers in an intermediate region between a top region of the convex portion and a bottom region of the concave portion is lower than the density of the fibers in each of the top region and the bottom region. This document mentions an example of using, as the surface sheet, the nonwoven fabric made of 100% cotton fibers, which are non-heat-fusible hydrophilic fibers.

Prior Art Document

Patent Document

Patent Document 1: JP 2016-112155 A (claim 1, paragraph 0070)

Disclosure of the Invention

Problems to be Solved by the Invention

Fluffing may become a problem for sheets that contact the user's skin, such as surface sheets for absorbent articles, or sheets that contact the user's clothing, such as backsheets for absorbent articles. Fluffing tends to give the user a sensation that the product is unsanitary. If the fluff is tangled into a fluff ball, or the fluff falls off a fabric, the texture of the fabric may be degraded or the adhesion of the fiber to clothing may be noticeable. The fluffing is more likely to occur when fibers are insufficiently entangled or bonded together.
The present disclosure has an object of providing a sheet for an absorbent article that is less likely to cause the fluffing and fluff falling and has excellent aesthetic appearance.

Means for Solving the Problems

The present disclosure provides a sheet for an absorbent article, including a nonwoven fabric containing cellulose-based fibers in an amount of more than 90% by mass, the nonwoven fabric being formed by entangling fibers,

wherein the nonwoven fabric includes a plurality of first entanglement portions spaced apart from each other and a plurality of second entanglement portions spaced apart from each other,
each of the first entanglement portions has a regular pattern formed by a plurality of regions having a high fiber density (hereinafter referred to as "high-density regions") and a plurality of regions having a low fiber density (hereinafter referred to as "low-density regions"), the regular pattern having substantially identical low-density regions arranged in a staggered or lattice manner,
each of the first entanglement portions extends along a longitudinal direction or a lateral direction of the nonwoven fabric,
each of the second entanglement portions extends along the longitudinal direction or the lateral direction of the nonwoven fabric,
the first entanglement portions and the second entanglement portions are alternately arranged,
a combined area of the first entanglement portions is 20% or more of the entire area of the nonwoven fabric,
a basis weight of the nonwoven fabric is more than 30 g/m$^2$, and
a fluff falling amount in the following test is 1.0 mg or less.

(Fluff Falling Amount Measurement Test)

a) A disk (of 70 mm in diameter and 350 g in weight) having its surface covered with an urethane foam (manufactured by Bridgestone Corporation, trade name: MOLTOPREN MF30, 5 mm in thickness) is attached to a rotating shaft so that the rotating shaft is positioned 20 mm away from the center of the disk.
b) An urethane foam that is of the same kind as the above-mentioned urethane foam is laid on a lower surface of the nonwoven fabric, and then the nonwoven fabric is fixed on a stand with an upper surface of the nonwoven fabric being an exposed surface.
c) The disk is placed on the nonwoven fabric. At this time, a load applied to the nonwoven fabric is only a self-weight of the disk.
d) The disk is revolved on the nonwoven fabric by rotating the rotating shaft. Four sets of the revolutions are performed such that each set includes two clockwise rotations and two counterclockwise rotations. The revolution rate at this time is approximately three seconds per revolution.
e) After the four sets of revolutions, the fibers falling off the nonwoven fabric and attached to the surface of the urethane foam covering the disk are collected.
f) The processes a) to e) mentioned above are performed on 30 pieces of the nonwoven fabrics, i.e., n = 30. The mass of the fibers falling off each of the 30 pieces of nonwoven fabrics is measured, and finally an average of the measured masses is referred to as a fluff falling amount.

Effects of the Invention

According to the present disclosure, the sheet for an absorbent article that is less likely to cause fluffing and has excellent aesthetic appearance can be obtained.

Brief Description of the Drawings

Figs. 1(A) and 1(B) are plan views schematically showing examples of nonwoven fabrics of a present embodiment in which substantially identical low-density regions are arranged in a staggered manner and a lattice manner, respectively, and Fig. 1(C) is a plan view schematically showing another example of a nonwoven fabric of the present embodiment in which two types of low-density regions, each type having substantially identical low-density regions, are arranged in a staggered manner.
Fig. 2 is a plan view for explaining a pitch and a spacing between substantially identical low-density regions arranged in the staggered manner.
Fig. 3 is a plan view showing first entanglement portions which are meandering in an example of the nonwoven fabric of the present embodiment.
Fig. 4 is a plan view showing first entanglement portions which meander and do not have a constant width in another example of the nonwoven fabric of the present embodiment.
Fig. 5 is a photograph showing the surface of a nonwoven fabric obtained in Comparative Example 3.

Mode for Carrying Out the Invention

(Circumstances Leading to the Present Embodiment)

Like a surface sheet of a sanitary napkin or a diaper, when a sheet for an absorbent article (hereinafter also referred to simply as a "sheet") is used as a member in direct contact with the skin or the like, the sheet may be constituted of a nonwoven fabric that contains a large amount of cellulose-based fibers. Examples of the cellulose-based fibers include natural cellulose-based fibers, such as cotton, and regenerated fibers, such as viscose rayon. These fibers are said to be gentle to the skin and delicate zones and to be less irritating. The cellulose-based fiber imparts unique tactile sensation and feeling to the sheet, particularly when it is a natural fiber. Thus, absorbent articles incorporating a sheet that uses cellulose-based fibers can be sold by positively displaying the usage of the cellulose-based fibers as an advantageous feature of the product.
Sheets that are in direct contact with the skin or the like, such as surface sheets, are often configured using a nonwoven fabric produced by a method of entangling fibers with a high pressure fluid stream (also referred to as a "spunlace method"). The spunlace method can give relatively soft nonwoven fabrics. However, the nonwoven fabric produced by the spunlace method has a problem of fluffing or fluff falling (fluff dropping off the fabric) due to fibrillation of the fibers and/or protrusion of the fiber end. Especially, natural fibers, such as cotton, do not have a constant fiber length, and the ends of the fibers with a short fiber length are likely to protrude, causing the fluffing and fluff falling. Methods of suppressing the fluffing and fluff falling of the nonwoven fabric produced by the spunlace method include a method of bonding fibers and a method of strongly entangling fibers.

In general, cellulose-based fibers do not have bonding properties. In order to suppress the fluffing and fluff falling of the nonwoven fabric using cellulose-based fibers by bonding, it is necessary to mix a thermal bonding fiber in the cellulose-based fibers and then to melt or soften the thermal bonding fiber by a thermal treatment to thereby form bonding points, or alternatively to form bonding points with a binder. However, the bonding points formed by the thermal bonding fiber or binder imparts a hard touch to the nonwoven fabric, degrading the unique tactile sensation of the cellulose-based fibers. When the cellulose-based fiber is a natural fiber, the appealing point is "natural". If a large amount of the thermal bonding fiber or binder is used to suppress the fluffing and fluff falling, this appealing point may be weakened.

In the case of suppressing the fluffing and fluff falling by enhancing the degree of mutual entanglement of the fibers, there is no need to use bonding, which does not cause any inconvenience due to the bonding as mentioned above. However, when the energy of the high pressure fluid stream increases, unevenness due to a difference between fiber densities occur, deteriorating the texture of the nonwoven fabric. The deterioration of the texture of the nonwoven fabric constituting the user-visible sheet, such as a surface sheet, may give the user an impression that the quality of an absorbent article is low. The smaller the basis weight of the nonwoven fabric is, the more pronounced the deterioration of the texture due to an increase in the energy of the high pressure fluid stream becomes.

The present inventors have studied about the structure of a sheet that contains a nonwoven fabric preferably composed of only cellulose-based fibers, has a good texture even at a small basis weight, and is less likely to cause the fluffing and fluff falling. As a result, the present inventors have found that the fluffing and fluff falling can be suppressed by the configuration of the nonwoven fabric in which entanglement portions with a regular pattern formed by portions having a high fiber density and portions having a low fiber density, and other entanglement portions having a relatively uniform fiber density are arranged to form a stripe pattern. The reason why the fluffing and fluff falling are suppressed by such a structure is considered to be due to the fact that when forming the regular pattern, the fibers in the entanglement portion are tightly entangled with each other.

The present inventors have also found that the fluffing and fluff falling is further suppressed by setting a ratio of the entanglement portions having the regular pattern in the nonwoven fabric within a predetermined range and also setting the basis weight of the nonwoven fabric larger than a predetermined value. Further, the present inventors have found that the nonwoven fabric having the specific regular pattern has improved water absorbability. In addition, the present inventors have also found that the nonwoven fabric with such a structure exhibits excellent design effect.

A sheet for an absorbent article of the present embodiment and an absorbent article using the same will be described below.

A sheet for an absorbent article of the present embodiment includes:

a nonwoven fabric containing cellulose-based fibers in an amount of more than 90% by mass, the nonwoven fabric being formed by entangling fibers,
wherein the nonwoven fabric includes a plurality of first entanglement portions spaced apart from each other and a plurality of second entanglement portions spaced apart from each other,
each of the first entanglement portions has a regular pattern formed by a plurality of regions having a high fiber density (hereinafter referred to as "high-density regions") and a plurality of regions having a low fiber density (hereinafter referred to as "low-density regions"), the regular pattern having substantially identical low-density regions arranged in a staggered or lattice manner,
each of the first entanglement portions extends along a longitudinal direction or a lateral direction of the nonwoven fabric,
each of the second entanglement portions extends along the longitudinal direction or the lateral direction of the nonwoven fabric,
the first entanglement portions and the second entanglement portions are alternately arranged,
a combined area of the first entanglement portions is 20% or more of the entire area of the nonwoven fabric,
a basis weight of the nonwoven fabric is more than 30 g/m$^2$, and
a fluff amount in the following test is 1.0 mg or less.

(Fluff Falling Amount Measurement Test)

a) A disk (of 70 mm in diameter and 350 g in weight) having its surface covered with urethane foam (manufactured by Bridgestone Corporation, trade name: MOLTOPREN MF30, 5 mm in thickness) is attached to a rotating shaft so that the rotating shaft is positioned 20 mm away from the center of the disk.

b) An urethane foam that is of the same kind as the above-mentioned urethane foam is laid on a lower surface of the nonwoven fabric, and then the nonwoven fabric is fixed on a stand with an upper surface of the nonwoven fabric being an exposed surface.

c) The disk is placed on the nonwoven fabric. At this time, a load applied to the nonwoven fabric is only a self-weight of the disk.

d) The disk is revolved on the nonwoven fabric by rotating the rotating shaft. Four sets of the revolutions are performed such that each set includes two clockwise rotations and two counterclockwise rotations. The revolution rate at this time is approximately three seconds per revolution.

e) After the four sets of revolutions, the fibers falling off the nonwoven fabric and attached to the surface of the urethane foam covering the disk are collected.

f) The processes a) to e) mentioned above are performed on 30 pieces of the nonwoven fabrics, i.e., n = 30. The mass of the fibers falling off each of the 30 pieces of nonwoven fabrics is measured, and finally an average of the measured masses is referred to as a fluff falling amount.

(Cellulose-Based Fiber)

The cellulose-based fiber contained in the nonwoven fabric included in the sheet for an absorbent article of the present embodiment (hereinafter also referred to simply as the "nonwoven fabric") will be described below.

Examples of the cellulose-based fiber used in the present embodiment include:

- natural fibers derived from plants, such as cotton, linen, ramie, jute, and hemp;
- rayon and polynosic obtained by a viscose method; cupra obtained by a copper-ammonia method; and regenerated fibers, such as cellulose fibers obtained by a solvent spinning method (Lyocell (registered trademark) and TENCEL (registered trademark) manufactured by Lenzing Co., Ltd.);
- cellulose fibers obtained by a melt spinning method; and
- semi-synthetic fibers, such as acetate fibers. In the present embodiment, the cellulose-based fiber is not particularly limited, and one or more kinds of these cellulose-based fibers may be arbitrarily selected.

In the present embodiment, particularly, cotton is used. Cotton is a natural fiber and is suitable for constituting a sheet that is in contact with the skin because it is less irritating to the skin. The cotton may have water repellency. The cotton having the water repellency may be, for example, cotton with a cotton wax attached thereto, or cotton from which a cotton wax is removed by scouring and bleaching and to which a water repellent is then attached.

The cotton wax is a natural wax attached to the cotton. By appropriately selecting the conditions for the scouring and bleaching treatment, a desired amount of cotton wax can be left in the cotton, thereby obtaining the desired water repellency. A method of attaching the water repellent to the cotton from which the cotton wax has been removed is a method that includes, for example, applying a commercially available water repellent to one surface of a nonwoven fabric produced by the spunlace method, and then drying the nonwoven fabric. The application of the water repellent may be performed by an arbitrary method, for example, using a gravure coater, a reverse roll coater, a kiss coater, or the like.

The cotton with the water repellency has the property of floating on the surface of water for a relatively long time when it is put into water because it is less likely to absorb water. In the present embodiment, the cotton that has enough water repellency not to sink for 30 seconds or more when it is put into water is preferably used as the cotton with the water repellency.

When the cotton with the water repellency is used, the resultant nonwoven fabric shows a smaller amount of liquid return. In general, when using water-repellent fibers, the liquid absorption rate, at which the nonwoven fabric absorbs the liquid, tends to decrease. However, the liquid absorption rate of the nonwoven fabric of the present embodiment that uses the water-repellent fiber can be the same as or higher than the liquid absorption rate of the nonwoven fabric of the present embodiment using a non-water repellent fiber. This is considered to be because the nonwoven fabric of the present embodiment has regions having a low fiber density. When the cotton with the water repellency is a cotton with a cotton wax attached thereto, the cotton wax imparts good tactile sensation to the nonwoven fabric.

The fineness of the cellulose-based fiber is not particularly limited, and may be, for example, in a range of 0.6 dtex to 5.6 dtex, particularly 1.4 dtex to 4.4 dtex, and more particularly 1.7 dtex to 3.3 dtex. If the fineness of the cellulose-based fiber is extremely small, the nonwoven fabric may become extremely soft, degrading the handleability. In contrast, when the fineness is extremely large, the tactile sensation of the nonwoven fabric may be degraded. The fineness of the natural fiber can be calculated in accordance with JIS L 1019 7.4.1 Micronaire method (the same applies below).

The fiber length of the cellulose-based fiber is not particularly limited and may be appropriately selected depending on a manufacturing method of a nonwoven fabric or the like. When a carded web is produced to manufacture a nonwoven fabric, the fiber length of the cellulose-based fiber may be, for example, in a range of 25 mm to 100 mm and in particular 30 mm to 70 mm. All the fiber lengths do not need to be the same. For example, natural fibers derived from plants, such as cotton, are generally supplied in the form wherein their fiber lengths are not constant. However, such a form may be used in the present embodiment.

(Other Fibers)

The nonwoven fabric may include fibers other than the cellulose-based fiber. The other fibers may be, for example, one or more fibers selected from natural fibers (e.g., wool, silk, etc.,) which are not cellulose-based fibers, and synthetic fibers composed of thermoplastic synthetic resins. When the other fibers are synthetic fibers, the thermoplastic resin is not particularly limited. The thermoplastic resin is optionally selected from polyester-based resins such as polyethylene terephthalate, polybutylene terephthalate, polytrimethylene terephthalate, polyethylene naphthalate, polylactic acid, polybutylene succinate and copolymers thereof; polyolefin-based resins such as polypropylene, polyethylene (including high-density polyethylene, low-density polyethylene, linear low-density polyethylene, and the like), polybutene-1, propylene copolymers mainly composed of propylene (including propylene-ethylene copolymers, and propylene-butene-1-ethylene copolymers), and ethylene-vinyl acetate copolymers; polyamide-based resins such as nylon 6, nylon 12, and nylon 66; acrylic resins; and engineering plastics such as polycarbonates, polyacetal, polystyrene, and cyclic polyolefins; and elastomers thereof.

The synthetic fiber may be a single fiber composed of one or more thermoplastic resins selected from the above, or may be a conjugate fiber composed of two or more components (also referred to as "sections"). In the conjugate fiber, each component may be composed of one thermoplastic resin or a mixture of two or more thermoplastic resins. The conjugate fiber may be, for example, a core-sheath type conjugate fiber, an island-in-sea type composite fiber, a splittable conjugate fiber, or a side-by-side type conjugate fiber. The core-sheath type conjugate fiber may be an eccentric core-sheath type conjugate fiber in which the center of a core component and the center of a sheath component do not coincide with each other in the fiber cross section, or a concentric core-sheath type conjugate fiber in which the center of the core component and the center of the sheath component coincide with each other in the fiber cross section.

The fineness of the other fibers is not particularly limited, and may be, for example, in a range of 1.4 dtex to 7.8 dtex, particularly 2.2 dtex to 6.7 dtex, and more particularly 3.3 dtex to 5.6 dtex. If the fineness of the fiber is too small, the nonwoven fabric may become extremely soft, degrading the handleability. In contrast, if the fineness thereof is too large, the tactile sensation on the nonwoven fabric may be degraded.

The fiber length of the other fibers is not particularly limited and may be appropriately selected depending on a manufacturing method of a nonwoven fabric or the like. When a carded web is produced to manufacture a nonwoven fabric, the fiber length of the other fibers may be, for example, in a range of 25 mm to 100 mm, and particularly 30 mm to 70 mm. All the fiber lengths of the other fibers do not need to be the same.

(Structure of Nonwoven Fabric)

The structure of the nonwoven fabric included in the sheet for an absorbent article of the present embodiment will be described below.

[Ratio of Fibers etc.]

The nonwoven fabric contains cellulose-based fibers in an amount of more than 90% by mass. The cellulose-based fibers are used to impart the unique tactile sensation and feeling to the nonwoven fabric and to make the nonwoven fabric less irritating. When the ratio of the cellulose-based fibers in the nonwoven fabric is equal to or less than 90% by mass, the nonwoven fabric may not give sufficient tactile sensation and feeling of the cellulose-based fibers and may irritate the skin more because the ratio of other fibers in the nonwoven fabric becomes large. The cellulose-based fibers may be contained in an amount of particularly 95% by mass or more and more particularly 100% by mass. When the nonwoven fabric is composed of the cellulose-based fibers only, the effect exhibited by using the cellulose-based fibers can be maximized.

When the nonwoven fabric contains fibers other than the cellulose-based fibers, the other fibers are contained at a ratio of 10% by mass or less in the nonwoven fabric. When the ratio of the other fibers exceeds 10% by mass, the effect conferred by the cellulose-based fibers may not be obtained sufficiently. The other fiber may be contained, particularly at a ratio of 5% by mass or less.

When the nonwoven fabric contains synthetic fibers as the other fibers, the fibers may be bonded together by the other fibers in the nonwoven fabric. Bonding of the fibers can suppress fluffing of the nonwoven fabric. However, the tactile sensation of the nonwoven fabric may become hard by the bonding, and thereby bonded portions may irritate the skin. For this reason, when using the nonwoven fabric as a member designed focusing on tactile sensation, such as a surface sheet, it is preferable that the fibers are not bonded together.

The fibers may not be sufficiently entangled with each other when the nonwoven fabric is produced by a manufacturing method using a high pressure fluid (particularly, high pressure water) to be mentioned later, in the case of using the cotton with the cotton wax attached. In such a case, to improve the entanglement of the fibers, the cotton from which the cotton wax is removed may be mixed. The cotton from which the cotton wax is removed may be contained in the nonwoven fabric at a ratio of 70% or less when the combined mass of this cotton and the cotton with the cotton wax attached is 100%.

[First Entanglement Portion and Second Entanglement Portion]

The nonwoven fabric include a plurality of first entanglement portions spaced apart from each other and a plurality of second entanglement portions spaced apart from each other.

The first entanglement portion is a portion where fibers are entangled with each other, and which extends along the longitudinal direction or lateral direction of the nonwoven fabric. As used herein, the expression "extending along the longitudinal direction or the lateral direction of the nonwoven fabric" means that the length direction of the first entanglement portion is parallel to the longitudinal direction (also referred to as a "MD direction") or the lateral direction (also referred to as a "CD direction") of the nonwoven fabric. Therefore, when the plurality of first entanglement portions extend, for example, along the longitudinal direction, they are arranged side by side along the lateral direction, whereas when the plurality of first entanglement portions extend, for example, along the lateral direction, they are arranged side by side along the longitudinal direction.

As mentioned later, the first entanglement portion may meander. Here, when the first entanglement portion is meandering between two straight lines parallel to the longitudinal direction of the nonwoven fabric, the first entanglement portion extends along the longitudinal direction of the nonwoven fabric. In this case, the length direction of the first entanglement portion is set as the longitudinal direction, for convenience. Similarly, when the first entanglement portion is meandering between two straight lines parallel to the lateral direction of the nonwoven fabric, the first entanglement portion extends along the lateral direction of the nonwoven fabric. In this case, the length direction of the first entanglement portion is set as the lateral direction, for convenience.

The first entanglement portions has low-density regions and high-density regions. In the first entanglement portion, the low-density regions form a regular pattern. As used herein, the term "regular pattern" refers to a pattern formed by arranging the high-density regions or the low-density regions according to a certain rule. The regular pattern can improve the aesthetic appearance of the nonwoven fabric. As mentioned later, the regular pattern can be formed by disposing a web on a support with regular arrangement of at least one selected from a convex portion, a concave portion, and an opening portion, and then by applying a high pressure fluid to the web.

The low-density region may be a portion that does not have a different thickness from that of the high-density region (i.e., the low-density region and the high-density region may not form any concavo-convex portion), or may be a concave portion or an opening portion. As mentioned later, the low-density regions are formed by, for example, placing a web on a fabric made of relatively thick monofilaments, and applying an entanglement treatment to the web using a high pressure fluid (high pressure gas, such as compressed air, high pressure liquid, such as high pressure water, or the like), to cause rearrangement of the fibers of the fibrous web located on crossing points of the fabric by moving the fibers toward the periphery of the web through a high pressure fluid action (hereinafter also referred to simply as "moving"). When the low-density regions are formed by such a method, an area per low-density region is determined mainly by the thickness of each yarn constituting the fabric. Alternatively, the low-density region may be formed with a molded body having at least one selected from a convex portion, a concave portion and an opening portion, or by a spiral net.

One low-density region may have an area of, for example, 0.03 mm$^2$ to 20 mm$^2$, particularly 0.1 mm$^2$ to 10 mm$^2$, and more particularly 0.7 mm$^2$ to 5.0 mm$^2$. If the low-density region is extremely small, the low-density region may not be sufficiently recognized, so that the design effect may not be sufficiently exhibited. If the low-density region is extremely large, the nonwoven fabric is prone to breakage or deformation, such as elongation, kink or break, which degrades the handleability of the nonwoven fabric. If the low-density region is extremely large, the low-density region may become difficult to recognize, and thus the design effect may not be sufficiently exhibited.

The low-density region is a region that has a smaller fiber density than that of at least a high-density region. The low-density region may have a fiber density of, for example, 0 g/cm$^3$ to 0.080 g/cm$^3$, particularly 0 g/cm$^3$ to 0.070 g/cm$^3$, and more particularly 0 g/cm$^3$ to 0.05 g/cm$^3$. The fiber density of zero means that the low-density region is an opening portion. When the fiber density of the low-density region is relatively large and a difference in the fiber density between the low-density region and the high-density region is small, the pattern becomes difficult to recognize, and the design effect may not be exhibited sufficiently.

In one first entanglement portion, or one nonwoven fabric, the fiber density, shape, and area of each low-density region is not necessarily the same. For example, within one first entanglement portion, one low-density region may be an opening portion, while another low-density region may be a region which is not an opening portion.

In the present embodiment, the regular pattern has substantially identical low-density regions arranged in a staggered or lattice manner (hereinafter also referred to as "low-density regions A"). As used herein, the term "substantially identical low-density regions" means the low-density regions having substantially the same shape and area. Alternatively, the term "substantially identical low-density regions" means regions resulting from the use of a support on which convex portions, concave portions or opening portions which have the same shape and area are formed regularly during the formation of the pattern by the high pressure fluid. In the present embodiment, for example, the low-density regions whose areas are different from each other by approximately 30%, particularly approximately 20%, can be regarded as the low-density regions A. When the low-density regions A have substantially the same shape and area, one low-density region A may be an opening portion, while the other low-density region A may be a concave portion.

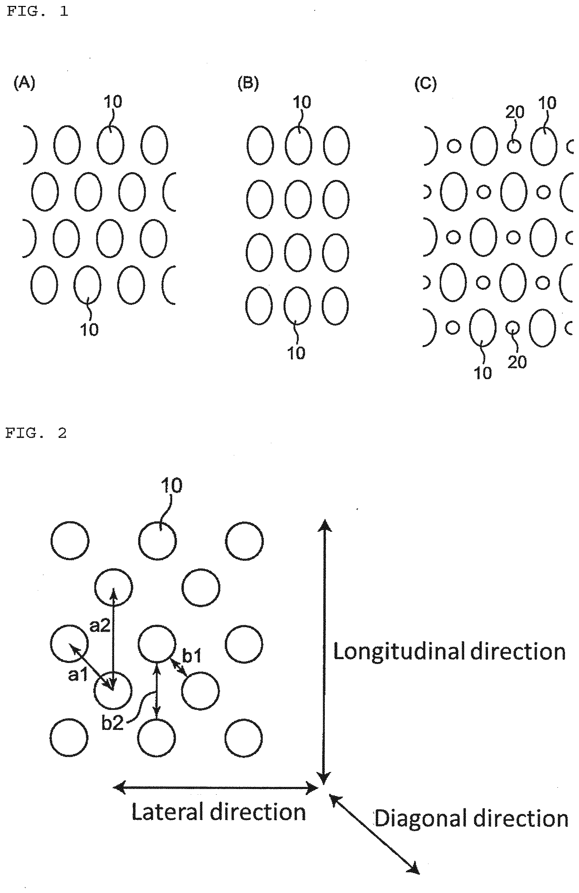

Fig. 1(A) and Fig. 1(B) are respective plan views schematically showing examples of the low-density regions A arranged in the staggered manner and the lattice manner, respectively. In Fig. 1(A) and Fig. 1(B), the low-density region A is denoted by reference numeral 10 and has an elliptical shape. The shape of the low-density region A is not limited thereto, and may be circular, polygonal (for example, triangular, rectangular, square, or rhombic), starshaped, or the like. In the present embodiment, two or more kinds of low-density regions A may be formed. For example, as shown in Fig. 1(C), the regular pattern may include elliptical low-density regions A denoted by 10 arranged in the staggered manner and circular low-density regions A denoted by20 arranged in a staggered manner.

In the present embodiment, one low-density region with a certain shape and dimension and all other low-density regions that are substantially identical and adjacent to the one low-density region have a relationship of either a staggered arrangement or a lattice arrangement therebetween. Therefore, in the present embodiment, it is not considered that the substantially identical low-density regions are formed in the staggered or lattice manner when a low-density region b adjacent to a certain low-density region a (and substantially identical to the low-density region a) has the relationship of the lattice arrangement with respect to the low-density region a, while another adjacent low-density region c (substantially identical to the low-density region a) has the relationship of the staggered arrangement with respect to the low-density region a.

As long as the low-density regions A are formed in the staggered or lattice manner, the regular pattern may have another kind of low-density region. For example, a low-density region with a different dimension and/or shape may be formed between the adjacent low-density regions A.

A pitch between the adjacent low-density regions A is not particularly limited and may be, for example, in a range of 0.1 mm to 50 mm, particularly 0.5 mm to 30 mm, and more particularly 1 mm to 10 mm in at least one direction. Here, the pitch corresponds to the distance between the centers of gravity of the adjacent low-density regions A in one first entanglement portion. An interval between the low-density regions A may be, for example, in a range of 0.1 mm to 50 mm, particularly 0.5 mm to 30 mm, and more particularly 1.0 mm to 10 mm in at least one direction. Here, the interval indicates the shortest line segment among line segments connecting an arbitrary point on an outer periphery (contour) of one low-density region A and an arbitrary point on an outer periphery (contour) of another low-density region A adjacent to the one low-density region A in one first entanglement portion.

In one first entanglement portion or one nonwoven fabric, the pitch and interval between the low-density regions A may not be constant. For example, when the low-density regions A denoted by10 are arranged in the staggered manner as shown in Fig. 2, the pitch (a1 and a2 in the figure) and the interval (b1 and b2 in the figure) vary depending on its direction. However, in the present embodiment, at least the shortest pitch and the shortest interval are preferably within the above-mentioned respective ranges. Further, the pitch and the interval in all directions are more preferably within the above-mentioned respective ranges.

In the present embodiment, the low-density regions A may be formed such that the sum of their areas (i.e., the total combined area of the low-density regions A in the nonwoven fabric) is, for example, in a range of 7.0% to 25%, particularly 7.5% to 20%, and more particularly 8.0% to 15% of the area of the entire nonwoven fabric. The ratio (area ratio) of the low-density regions A within this range, along with the arrangement of the low-density regions in the staggered manner or lattice manner, increases the liquid absorption rate of the nonwoven fabric.

The high-density region is formed between the adjacent low-density regions or around the low-density region. As mentioned above, the high-density region is a region where fibers moved by the high pressure fluid action are densely aggregated when forming the low-density regions.

The high-density region has a higher fiber density than the low-density region. The high-density region may have a fiber density of, for example, 0.01 g/cm$^3$ to 0.30 g/cm$^3$, particularly 0.03 g/cm$^3$ to 0.20 g/cm$^3$, and more particularly 0.05 g/cm$^3$ to 0.15 g/cm$^3$. The high-density region may have a thickness of, for example, 0.05 mm to 5.0 mm, particularly 0.10 mm to 3.0 mm, and more particularly 0.2 mm to 2.0 mm. Here, the fiber density is determined by: the thickness of the nonwoven fabric measured with no load applied to the nonwoven fabric; and the basis weight of the nonwoven fabric. The thickness of the nonwoven fabric with no load applied to the nonwoven fabric can be determined, for example, by observing an electron micrograph of the nonwoven fabric.

The width of each of the first entanglement portions may be, for example, in a range of 3 mm to 200 mm, particularly 3 mm to 50 mm, more particularly 5 mm to 50 mm, still more particularly from 5 mm to 30 mm, or alternatively from 5 mm to 15 mm. The width of the first entanglement portion refers to a dimension of the first entanglement portion in a direction orthogonal to the direction in which the first entanglement portion extends. In the one first entanglement portion, its width may or may not be constant.

In the nonwoven fabric, first entanglement portions with different widths may be present. For example, first entanglement portions having a narrow width (for example, of 2 mm to 5 mm) and first entanglement portions having a wide width (for example, of 6 mm to 15 mm) may be alternately arranged. Alternatively, a first entanglement portion having a narrow width (for example, of 2 mm to 3 mm), a first entanglement portion having a medium width (for example, of 4 mm to 5 mm), and a first entanglement portion having a wide width (for example, 6 mm to 15 mm) may be arranged in the order of narrow-medium-wide or narrow-wide-medium.

The combined area of the first entanglement portions in the nonwoven fabric is 20% or more, preferably 25% or more, more preferably 30% or more, and most preferably 40% or more of the entire nonwoven fabric. Since the fibers are relatively tightly entangled with each other in the first entanglement portions, the first entanglement portions are portions that serve to suppress the fluffing and fluff falling of the nonwoven fabric. Therefore, if the ratio of the first entanglement portions in the nonwoven fabric is small, the fluffing and fluff falling are more likely to occur. The upper limit of the ratio of the combined area of the first entanglement portions in the nonwoven fabric is, for example, 65%, particularly 60%, more particularly 55%, and still more particularly 53%. If the ratio of the combined area of the first entanglement portions is extremely large, the amount of liquid return may become large when using the nonwoven fabric as a surface sheet for an absorbent article or the strength of the nonwoven fabric may be reduced.

In the nonwoven fabric, the first entanglement portion may be linear or meandering.

When the first entanglement portion is meandering, the length of the meandering per cycle (i.e., the wavelength thereof) may be 5 mm or more. The length of the meandering per cycle is determined as follows.

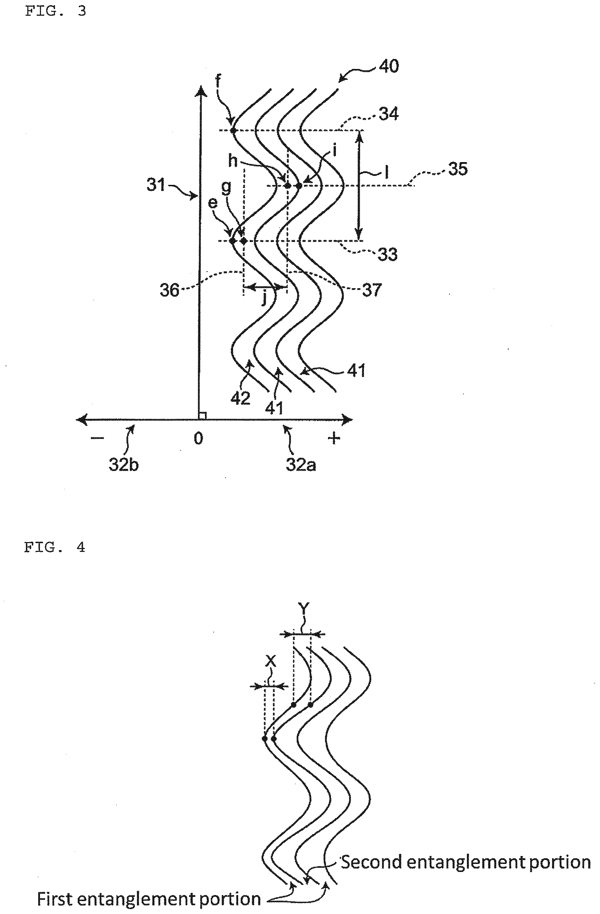

i) As shown in FIG. 3, the direction parallel to a straight line that is orthogonal to a longitudinal direction 31 of a first entanglement portion 42 is referred to as an orthogonal direction; the direction extending to the "+" side is referred to as a positive orthogonal direction 32a; and the direction extending to the "-" side is referred to as a negative orthogonal direction 32b.

ii) A straight line 33 orthogonal to the longitudinal direction 31 is drawn at a point e where the meandering progress of the first entanglement portion 42 proceeding in the negative orthogonal direction 32b changes its direction to the positive orthogonal direction 32a (while paying attention to the progress of one end portion in the width direction (left end portion in the figure) because the first entanglement portion 42 has a width).

iii) A straight line 34 orthogonal to the longitudinal direction 31 is drawn at a point f where the meandering progress of the first entanglement portion 42 which has proceeded from the point e in the positive orthogonal direction 32a and then progressed in the negative orthogonal direction 32b, changes its direction to the positive orthogonal direction 32a.

iv) A distance 1 between the straight line 33 and the straight line 34 is regarded as the length of the meandering progress per cycle. In points where the meandering progress of the first entanglement portion 42 changes its direction from the negative orthogonal direction 32b to the positive orthogonal direction 32a, for example, portions of the first entanglement portion 42 may proceed straightly in the longitudinal direction 31 (for example, when it is meandering in the form of a rectangular wave). In this case, straight lines 33 and 34 orthogonal to the longitudinal direction 31 are drawn at the midpoints of the straight portions of the first entanglement portion 42. In FIG. 3, reference numeral 41 denotes a second entanglement portion formed between the first entanglement portions 42.

In the present embodiment, the upper limit of the wavelength may be 200 mm. The wavelength is particularly in a range of 10 mm to 150 mm, and more particularly 30 mm to 100 mm. This makes it possible for the meandering pattern to be visually recognized clearly, and for the ratio of the combined area of the first entanglement portions to be optimized, when using the nonwoven fabric as the surface sheet for an absorbent article.

The meandering of the first entanglement portion preferably has an amplitude of 1 mm or more. The amplitude is determined as follows.

i) As shown in FIG. 3, a point on the above-mentioned straight line 33 that equally divides the width of the first entanglement portion 42 is denoted by g.

ii) A straight line 35 orthogonal to the longitudinal direction 31 is drawn at a point i where the meandering progress of the first entanglement portion 42 proceeding toward the positive orthogonal direction 32a changes its direction to the negative orthogonal direction 32b, and then a point on the drawn straight line 35 that equally divides the width of the first entanglement portion 42 is denoted by h.

iii) The amplitude is defined as a distance j between the straight line 36 including the point g and parallel to the longitudinal direction 31 and the straight line 37 including the point i and parallel to the longitudinal direction.

The upper limit of the amplitude may be 200 mm. The amplitude is particularly in a range from 2 mm to 150 mm, more particularly from 5 mm to 100 mm, and still more particularly from 10 mm to 50 mm.

The length per cycle of the meandering of the first entanglement portion may vary for each cycle, or the amplitude of each cycle may vary, like an attenuation wave. Alternatively, such a meandering cycle may be repeated regularly. The preferable configuration of the meandering, is one wherein the length per cycle of the meandering is the same for each cycle, and the amplitude of each cycle is also be the same, that is, the repeat of the same pattern.

A value of the ratio of the length (wavelength) per cycle of the meandering to the amplitude thereof (i.e., wavelength/amplitude) may be, for example, in a range of 1 to 15, particularly 1.5 to 12 and more particularly 2 to 8. When the value of the ratio of the wavelength to the amplitude is less than 1 or exceeds 15, the meandering of the first entanglement portion may become difficult to recognize. Fig. 3 shows an example in which the phases of meandering of the adjacent first entanglement portions are the same. The phases of the meandering of the adjacent first entanglement portions may deviate from each other.

When the first entanglement portions are meandering and the width of one first entanglement portion is not constant as shown in Fig. 4, the width X at a turn-around point of the meandering (hereinafter referred to simply as the "width X") may be different from the width Y of the other portions of the meandering (hereinafter referred to simply as the "width Y"). For example, the width X may be smaller than the width Y.

The second entanglement portion is an entanglement portion independent from the first entanglement portion, and the state of entanglement of fibers in the second entanglement portion differs from the state of entanglement of fibers in the first entanglement portion. In the present embodiment, the first entanglement portions and the second entanglement portions are alternately arranged. Therefore, the second entanglement portion forms a stripe pattern together with the first entanglement portion (a meandering stripe pattern when the first entanglement portion is meandering).

The second entanglement portion does not have any pattern, i.e., is plain. When the second entanglement portion is formed by the entanglement treatment using the high-pressure fluid stream, thin streaks may be formed at locations on the second entanglement portions where the high-pressure fluid stream hits. However, the second entanglement portions with such streaks are regarded as the plain.

If the second entanglement portion does not have a pattern and has a relatively uniform fiber density, the liquid return is further suppressed when using the nonwoven fabric as the surface sheet for an absorbent article. The term "liquid return" means that the liquid having passed through the surface sheet exudes to the surface of the surface sheet again. If the second entanglement portion also has a pattern, the high-density regions are present across the entire nonwoven fabric. Since the high-density region is a portion that can easily retain the liquid, and the retained liquid is easily pushed back by pressurization, the amount of liquid return in the nonwoven fabric may become large if the high-density regions are present across the entire nonwoven fabric.

The second entanglement portion may have a higher fiber density than the low-density region of the first entanglement portion and may have a lower fiber density than the high-density region of the first entanglement portion. The second entanglement portion may have, for example, a fiber density of 0.01 g/cm$^3$ to 0.20 g/cm$^3$, particularly 0.03 g/cm$^3$ to 0.15 g/cm$^3$, and more particularly 0.05 g/cm$^3$ to 0.13 g/cm$^3$.

The second entanglement portion may have, for example, a thickness of 0.05 mm to 3.0 mm, particularly 0.1 mm to 2.5 mm, and more particularly 0.2 mm to 1.8 mm.

When the low-density region in the first entanglement portion is an opening portion, the ratio of the fiber density of the second entanglement portion to the fiber density of the high-density region in the first entanglement portion (the second entanglement portion/the high-density region of the first entanglement portion) may be 1/(1.03 or more) and preferably 1/(1.05 or more). When the low-density region in the first entanglement portion is not an opening portion, the ratio of the fiber density of the low-density region in the first entanglement portion to the fiber density of the second entanglement portion (the low-density region of the first entanglement portion/the second entanglement portion) may be, for example, 1/(1.03 or more), and particularly 1/(1.05 or more). When a difference in the density between the low-density region of the first entanglement portion and the second entanglement portion, and/or a difference in the density between the second entanglement portion and the high-density region of the first entanglement portion is small, the first entanglement portion and the second entanglement portion may not be clearly distinguished from each other, whereby the design effect may not be sufficiently exhibited.

The width of each of the second entanglement portions may be, for example, in a range of 3 mm to 200 mm, particularly 3 mm to 50 mm, more particularly 5 mm to 50 mm, still more particularly 5 mm to 30 mm, or alternatively from 5 mm to 15 mm. The width of the second entanglement portion refers to a dimension of the second entanglement portion in a direction orthogonal to the longitudinal direction thereof. In the nonwoven fabric, second entanglement portions with different widths may be present. For example, second entanglement portions having a narrow width (for example, of 2 mm to 5 mm) and second entanglement portions having a wide width (for example, of 6 mm to 15 mm) may be alternately arranged. Alternatively, a second entanglement portion having a narrow width (for example, of 2 mm to 3 mm), a second entanglement portion having a medium width (for example, of 4 mm to 5 mm), and a second entanglement portion having a wide width (for example, 6 mm to 15 mm) may be arranged in the order of narrow-medium-wide or narrow-wide-medium.

When all the first entanglement portions have the same width and all the second entanglement portions have the same width in the nonwoven fabric, the width of the first entanglement portion and the width of the second entanglement portion may be the same or different from each other. When the first entanglement portion has the same width as the second entanglement portion, the ratio of the combined area of the first entanglement portions in the nonwoven fabric is 50%. When the first and second entanglement portions have different widths, the width ratio of the first entanglement portion relative to the second entanglement portion may be, for example, 0.25 or more, particularly 0.5 or more, and more particularly 0.9 or more.

The second entanglement portions having different widths may be combined with the first entanglement portions having different widths. The first entanglement portions and the second entanglement portions may be alternately arranged side by side, for example, in the manner of wide(first entanglement portion, hereinafter referred to as just "first")-wide(second entanglement portion, hereinafter referred to as just "second")-narrow(first)-narrow(second). The first entanglement portions and the second entanglement portions may be alternately arranged side by side, for example, in the manner of wide(first)-wide(second)-medium(first)-medium(second)-narrow(first)-narrow(second) . Alternatively, the first entanglement portions and the second entanglement portions may be alternately arranged side by side, for example, in the manner of narrow(first)-narrow(second)-narrow (first)-wide(second). Here, the terms "wide", "medium", and "narrow" are used to indicate the magnitude relationship among the widths of the entanglement portions of the same kind. Therefore, the "wide" width of the first entanglement portion and the "wide" width of the second entanglement portion may not be the same. The combination of wide(first)-wide(second)-narrow(first)-narrow(second) includes, for example, a combination of 7 mm (first)-5 mm (second)-5 mm (first)-3 mm (second).

When the nonwoven fabric has only the first entanglement portions and the second entanglement portions while the first entanglement portions are meandering, the second entanglement portion adjacent between the first entanglement portions is also meandering. In such a case, the length of the meandering per cycle and the amplitude of the second entanglement portion are determined by those of the first entanglement portion. Therefore, the description of the length of the meandering per cycle and the amplitude of the second entanglement portion is omitted here.

[Basis Weight and so on]

The nonwoven fabric constituting the sheet for an absorbent article of the present embodiment has a basis weight of more than 30 g/m². When the basis weight of the nonwoven fabric is 30 g/m² or less, the fluffing and fluff falling is more likely to occur, and the texture of the nonwoven fabric tends to be deteriorated. The basis weight of the nonwoven fabric may be, for example, 32 g/m² or more and 60 g/m² or less, particularly 33 g/m² or more and 50 g/m² or less, and more particularly 34 g/m² or more and 45 g/m² or less. If the basis weight of the nonwoven fabric is extremely large, the liquid is more likely to be retained in the nonwoven fabric. This may result in a larger amount of liquid return, when using the nonwoven fabric as the surface sheet. If the basis weight of the nonwoven fabric is extremely large, the air permeability thereof may be degraded.

The specific volume of the nonwoven fabric is not particularly limited, and is optimally selected in accordance with its application and the like. The specific volume of the nonwoven fabric may be, for example, 0.5 cm³/g or more and 2.0 cm³/g or less, particularly 0.7 cm³/g or more and 1.6 cm³/g or less, and more particularly 0.8 cm³/g or more and 1.5 cm³/g or less. The specific volume can be determined from the basis weight and thickness of the nonwoven fabric. Here, the thickness of the nonwoven fabric is measured while a load of 2.94 cN per cm² of a sample is applied to the sample. As the specific volume of the nonwoven fabric is larger, the air permeability and cushioning property of the nonwoven fabric tends to be improved. However, if the specific volume is extremely large, the liquid is more likely to be retained in the nonwoven fabric. This may result in a large amount of liquid return, when using the nonwoven fabric as the surface sheet.

[Fluff Falling Amount]

The nonwoven fabric constituting the sheet for an absorbent article in the present embodiment has a plurality of first entanglement portions that have a regular pattern, thereby making it less likely to cause the fluffing. Specifically, the nonwoven fabric is provided in which a fluff falling amount measured by the following method is 1.0 mg or less.

(Fluff Falling Amount Measurement Test)

**[0077]**

　　a) A disk (of 70 mm in diameter and 350 g in weight) that has its surface covered with urethane foam (manufactured by Bridgestone Corporation, trade name: MOLTOPREN MF30, 5 mm in thickness) is attached to a rotating shaft so that the rotating shaft is positioned 20 mm away from the center of the disk.

b) An urethane foam that is of the same kind as the above-mentioned urethane foam is laid on a lower surface of a nonwoven fabric, and then the nonwoven fabric is fixed on a stand with an upper surface of the nonwoven fabric being an exposed surface.

c) The disk is placed on the nonwoven fabric. At this time, a load applied to the nonwoven fabric is only the self-weight of the disk.

d) The disk is revolved on the nonwoven fabric by rotating the rotating shaft. Four sets of the revolutions are performed such that each set includes two clockwise rotations and two counterclockwise rotations. The revolution rate at this time is approximately three seconds per revolution.

e) After the four sets of revolutions, the fibers falling off the nonwoven fabric and attached to the surface of the urethane foam covering the disk are collected.

f) The processes a) to e) mentioned above are performed on 30 pieces of the nonwoven fabrics, i.e., n = 30. The mass of the fibers falling off each of 30 pieces of nonwoven fabrics is measured, and finally an average of the measured masses is referred to as a fluff falling amount.

The fluff amount may be particularly 1.0 mg or less, more particularly 0.8 mg or less, and still more particularly 0.5 mg or less. The nonwoven fabric that has a small fluff falling amount is a nonwoven fabric where the fluffing and fluff falling are less likely to occur, thus giving an impression of cleanliness to the user. If fluff is hardly generated in the nonwoven fabric, the generation of fluff balls due to tangling of the fluff is also suppressed, thereby giving smooth tactile sensation to the user. The nonwoven fabric that has a small fluff falling amount can reduce the amount of fibers attached to clothing when it comes into contact with the clothing of the user.

[Liquid Absorption Properties]

The liquid absorption rate of the nonwoven fabric constituting the sheet for an absorbent article of the present embodiment is evaluated using a run-off value in a direction orthogonal to an extending direction of the first entanglement portion and the second entanglement portion. Consequently, this nonwoven fabric exhibits a higher liquid absorption rate, compared to a liquid absorption rate in a nonwoven fabric that does not have the first entanglement portion and a nonwoven fabric that does not have the second entanglement portion. In addition, the nonwoven fabric constituting the sheet for an absorbent article of the present embodiment exhibits the higher liquid absorption rate, compared to a nonwoven fabric that does not have low-density regions A with a regular pattern arranged in a staggered or lattice manner.

Specifically, the run-off value is measured by the following procedure.

(i) A sample is prepared.

(ii) A slope that forms an angle of 30 degrees with respect to a horizontal plane is prepared. Two sheets of filter paper (Lister Paper (Grade 989, each 10 cm $\times$ 10 cm) are stacked on the slope and prepared as an absorber. Specifically, the absorbers are laid over an area of the slope that is equal to or greater than the area of the sample so that they do not overlap each other. The sample is placed and fixed onto the absorbers. The sample is disposed on the slope such that the extending directions of the first entanglement portion and the second entanglement portion are orthogonal to an inclination direction of the slope.

(iii) Then, 1.0 g or 3.0 g of 0.90% saline (colored with blue dyes) at 37°C was dropped at a position of 1cm below an upper end of the sample from a microtube pump or burette at a rate of 1.0 g/30 sec. When the whole saline is absorbed in the nonwoven fabric sample and droplets of the saline disappear from the surface of the nonwoven fabric sample, the position of the tip of the saline is measured. The run-off value is obtained by determining a distance between the above-mentioned position and a position on a surface of the nonwoven fabric sample at which the saline has been dropped, i.e., the longest distance by which the droplets of the saline have flowed on the surface of the nonwoven fabric sample.

Three samples are prepared for one nonwoven fabric. The values obtained by repeating a set of the processes (i) to (iii) three times are averaged, and the averaged value is used for evaluation as a run-off value of the nonwoven fabric.

The shorter the run-off value, the higher the liquid absorption rate is said to be. In the present embodiment, the nonwoven fabric has a structure in which the first entanglement portions and the second entanglement portions are alternately arranged. Thus, when liquid flows along the direction orthogonal to the extending direction of the first entanglement portion and the second entanglement portion, it is thought that the progress of the liquid is interrupted by a boundary between the two entanglement portions, thus making it easier for the liquid to progress along the thickness direction of the nonwoven fabric. When the low-density region and all other identical low-density regions adjacent thereto have the relationship of either the staggered arrangement or the lattice arrangement, the liquid regularly passes alternately through the low-density region and the high-density region in the first entanglement portion when the liquid passes on the first entanglement portion. Consequently, it is presumed that the rate at which the liquid flows changes regularly, which can further reduce the liquid absorption rate.

(Manufacturing Method of Nonwoven Fabric)

The nonwoven fabric is manufactured by a manufacturing method which includes: for example,

producing a fiber web containing 90% by mass or more of cellulose-based fibers;
applying an entanglement treatment to an entire fiber web using a high pressure fluid stream; and
forming a first entanglement portion having a regular pattern by partially applying an entanglement treatment using a high pressure fluid.

In the nonwoven fabric obtained by this manufacturing method, parts of the nonwoven fabric where no first entanglement portions are formed become the second entanglement portions. The entanglement treatment performed using the high pressure fluid stream for forming the first entanglement portions includes: disposing the fiber web on a support having at least one selected from a convex portion, a concave portion and an opening portion for forming the low-density regions (hereinafter referred to as a "pattern forming support"); and causing the high pressure fluid to act only on portions of the web where the first entanglement portions are to be formed. In each first entanglement portion, the low-density regions corresponding to the shape of the pattern forming support are formed, while the high-density regions are formed by the fibers moved to around the low-density regions between the adjacent low-density regions when forming the low-density regions.

Examples of the high pressure fluid include a high pressure gas, such as compressed air, and a high pressure liquid, such as high pressure water. The high pressure liquid is preferably used in terms of obtaining a nonwoven fabric with excellent tactile sensation and of being able to remove an excess oil agent attached to the fibers. In the manufacturing of the nonwoven fabric, a hydroentanglement treatment using high pressure water as the high pressure fluid is often employed. Also in the present embodiment, the hydroentanglement treatment is preferably used from the viewpoint of the easiness of implementation or the like. The manufacturing method using high pressure water (hereinafter, also referred to simply as "water stream") as the high pressure fluid will be described below.

First, the hydroentanglement treatment for entangling the fibers (hereinafter referred to as a first hydroentanglement treatment) is performed across the entire fibrous web. The first hydroentanglement treatment is performed by jetting a water stream toward the web placed on the support. The water stream may be a columnar water stream. As mentioned above, since the second entanglement portion is a portion in which the first entanglement portion is not formed, the first hydroentanglement process is to form the second entanglement portion. The second entanglement portion preferably has a uniform texture with less unevenness of fiber density. Therefore, the first hydroentanglement treatment preferably uses the support made of a relatively fine mesh, for example, a plain weave support with 80 mesh to 100 mesh.

The first hydroentanglement treatment may be performed by jetting a water stream at a water pressure of 1 MPa to 15 MPa onto each of the front and back surfaces of the web once to five times from a nozzle that has orifices of 0.05 mm to 0. 5 mm in diameter provided at intervals of 0.3 mm to 1.5 mm. The water pressure is preferably in a range of 1 MPa to 10 MPa, and more preferably 1 MPa to 7 MPa. The distance between the nozzle and the web may be, for example, in a range of 5 mm to 100 mm, and particularly 10 mm to 50 mm.

Alternatively, the first hydroentanglement treatment may be performed by selecting the water pressure, the number of jetting, the transfer speed, and the like so that the total energy (E) applied to the web by the high pressure water is in a range of 25 Wh/kg/m to 100 Wh/kg/m. E is determined by the following formula:

$$E = W \times N \times T/(M \times U \times 60)$$

where

E is the energy applied per kilogram of the non-woven fabric with 1 m in width for one hour (Wh/kg/m),
W is power (W) of fluid per orifice of the nozzle,
N is the number of orifices opened per meter of the nozzle,
T is the number of jets,
M is the basis weight ($g/m^2$) of an object to be treated by the high speed water stream, and
U is the transfer speed (m/min).

In the above-mentioned formula, W (the power of the fluid per orifice of the nozzle) is determined by the following formula:

$$W = P1 \times (F/100) \times 0.163)$$

where

W is a power (W) of fluid per orifice of the nozzle,
P1 is a water pressure ($kfg/cm^2$), and
F is a flow rate ($cm^3$/minute) of water discharged from one of the orifices of the nozzle.

The details of a method for determining E and W are mentioned in JP 4893256 B1.

Next, the hydroentanglement treatment for forming the first entanglement portion (hereinafter referred to as a second hydroentanglement treatment) is performed. When forming the first entanglement portion, as mentioned above, a web obtained after the first hydroentanglement treatment (hereinafter also referred to as a "primarily processed web") is disposed on a pattern formation support, and the water stream is applied only to parts of the web where the first entanglement portions are to be formed. Methods of partially applying the water stream to the web include, for example, a method of using a nozzle in which orifice groups each of which is consisted of one or more orifices, are provided at predetermined intervals, and a method of disposing a member having bores and impermeable to a high pressure fluid at any positions other than the bores (hereinafter simply referred to as a "bore member"), between the nozzle and the web.

The pattern formation support may have convex portions. In this case, when the water stream hits the fibers located on the convex portions, the fibers moves toward the peripheries of the convex portions, so that the fibers are rearranged to form the low-density regions at positions corresponding to the convex portions.

Alternatively, the pattern formation support may have concave portions. In this case, the fibers located around the peripheries of the concave portions move into the concave portions and are rearranged within the concave portions, so that the high-density regions are formed in the positions corresponding to the concave portions while the low-density regions are formed around the concave portions. The high-density regions can be three-dimensional depending on the shape of the concave portion.

Alternatively, the pattern formation support may have an opening portion. In this case, fibers located around the peripheries of the opening portions move into the opening portions and are rearranged within the opening portions, so that the high-density regions are formed in the positions corresponding to the opening portions while the low-density regions are formed around the opening portions. The high-density regions can be three-dimensional depending on the shape of the opening portion or the like. The pattern formation support may have portions of two or more types selected from the convex portion, the concave portion, and the opening portion.

The pattern formation support may be a fabric, a punched plate-shaped member, or a spiral net, which is made of a natural resin, a synthetic resin, or metal. The pattern formation support may have a regular pattern formed by regularly arranging one or more portions selected from a convex portion, a concave portion, and an opening portion. The use of such a pattern formation support can form the regular pattern by aggregating a plurality of low-density regions. To form the low-density regions A arranged in a staggered or lattice manner, it is preferable to use the pattern formation support with convex portions having substantially the same shape and area regularly arranged, in particular, in the staggered or lattice manner.

Specifically, the pattern formation support may be, for example, a plain weave fabric, a herringbone weave fabric, a twill weave fabric, or a satin weave fabric in which monofilaments with a fiber diameter of approximately 0.1 mm to 1.2 mm are woven at a warp yarn density of 10 lines/inch to 30 lines/inch and a weft yarn density of 10 lines/inch to 30 lines/inch. The support composed of a fabric of relatively thick filaments has convex portions at the crossing points of the weft and warp yarns, thus enabling the formation of the low-density regions. When using such a fabric, an area of one low-density region is determined depending on the thickness of the yarn constituting the fabric, and an interval and pitch between the low-density regions are determined depending on the warp yarn density and weft yarn density of the fabric.

In particular, the plain weave fabric composed of woven warp yarns and weft yarns, each yarn being a single monofilament, is preferably used. In such a plain weave fabric, since the highest portions of the crossing points between the warp and weft yarns become convex portions, which are arranged in a staggered manner, the use of the plain weave fabric can form the low-density regions A which are arranged in the staggered manner while corresponding to the above-mentioned portions.

Alternatively, the pattern formation support may include a convex portion and/or concave portion, for example, a frustoconical, a conical, a truncated pyramidal or a pyramidal projection, or a concave portion formed by machining or the like of the surface of a metal plate, and a portion of the pattern formation support other than the convex portion and/or concave portion may be a plate-shaped member (for example, a metal plate) that has, for example, a small sized opening to ensure to have water permeability.

Alternatively, the pattern formation support may be a spiral net.

In the method of forming the first entanglement portion using a nozzle provided with orifice groups spaced at predetermined intervals, only the water stream jetted from the orifice group acts on the web. Thus, the high pressure water stream with energy required to form the regular pattern acts only on the portions of the web corresponding to the orifice groups. The orifice group is provided across a section of the nozzle that corresponds to the width of the first entanglement portion. The interval between the adjacent orifice groups corresponds to the width of the second entanglement portion. Such a nozzle may be a nozzle designed to apply a water stream over its entire surface, wherein the orifices located in a section corresponding to the second entanglement portion are closed with stoppers. One orifice group preferably includes two or more orifices. When the number of orifices is 2 or more, the regular pattern can be formed more clearly in the first entanglement portion. The interval between adjacent orifices in the orifice group may be, for example, 0.2 mm to 1.5 mm.

In the method of forming the first entanglement portions using the bore member, only the water stream jetted through the bores acts on the web. Thus, the high pressure water stream with energy required to form the regular pattern acts only on the portions of the web corresponding to the bores. The bore member is not particularly limited as long as it is a member with a plurality of bores. For example, the material of the bore member may be a synthetic resin, metal, or the like. The shape of the bore member may be appropriately selected as, for example, a plate shape, a roll shape, or the like, in accordance with a hydroentanglement treatment device.

The plurality of bores included in the bore member are formed along the direction orthogonal to the direction in which the first entanglement portion extends. One bore may have, for example, a dimension of 2 mm or more, particularly a dimension of 3 mm to 50 mm, and more particularly a dimension of 5 mm to 30 mm, in the direction orthogonal to the direction in which the first entanglement portion extends. When forming the first entanglement portions having different widths, bores having different dimensions are formed in one bore member in accordance with the width of the first entanglement portion to be obtained. The interval between adjacent bores included in the bore member may be, for example, in a range of 2 mm or more, particularly 3 mm to 50 mm, and more particularly 5 mm to 30 mm. The interval between the adjacent bores determines the width of the second entanglement portion. Thus, the interval between the adjacent bores is appropriately selected in accordance with the width of the second entanglement portion to be obtained. The shape of the bore is not particularly limited, and may be, for example, a circle, a semicircle, an ellipse, a polygon such as a triangle or a quadrangle, a star polygon, a cross, a slit shape such as a straight line or a curved line, or the like.

When using the bore member, the nozzle is not particularly limited, and any nozzle that is of the same type as one described in relation to the first hydroentanglement treatment may be used. The distance between the bore member and the nozzle may be, for example, 1 mm or more. If the distance between the bore member and the nozzle is less than 1 mm, the bore member and the nozzle may come into contact with each other, whereby one or both of them may be damaged. The distance between the bore member and the nozzle may be, for example, 30 mm or less. If the distance between the bore member and the orifice exceeds 30 mm, the energy of the water stream may be reduced, failing to form the regular pattern well. The distance between the bore member and the primarily treated web may be, for example, in a range from 5 mm to 50 mm. If the distance between the bore member and the primarily treated web exceeds 50 mm, the energy of the water stream may be reduced, failing to form a regular pattern well.

The range of water pressure that may be adopted in the second hydroentanglement treatment is as described in relation to the first hydroentanglement treatment. The first entanglement is usually formed by jetting a water stream onto one surface of the primarily treated web only once. The pressure in the second hydroentanglement treatment may be particularly in a range of 1 MPa to 15 MPa, and more particularly 2 MPa to 10 MPa.

Alternatively, the second hydroentanglement treatment may be performed by selecting the water pressure, the number of jetting, the transfer speed (the speed of the pattern formation support), and the like so that the total energy (E) applied to the primarily treated web by the high pressure water is in a range of 20 Wh/kg/m to 200 Wh/kg/m. The way to determine E is as described above in relation to the first hydroentanglement treatment.

The second hydroentanglement treatment can give a nonwoven fabric having the structure in which the plurality of first entanglement portions and the plurality of second entanglement portions extend straightly in either the longitudinal direction or the lateral direction of the nonwoven fabric, by fixing the position of the nozzle or the bore member and moving the pattern formation support along the longitudinal direction or lateral direction of the primarily treated web (usually in the longitudinal direction).

Alternatively, the second hydroentanglement treatment can give a nonwoven fabric having the structure in which the first entanglement portions and the second entanglement portions are meandering, by vibrating the nozzle or bore member and moving the pattern formation support along the longitudinal direction or lateral direction of the fiber web (usually in the longitudinal direction). As used herein, the term "vibrating" means that the nozzle or bore member is reciprocating along a certain direction. The term "vibrating" includes, in addition to the case of reciprocating on a straight line, a case of reciprocating along an elliptical orbit which has a long axis in a certain direction, and the like.

The vibrating direction of the nozzle or bore member may be appropriately selected from the longitudinal direction (MD direction), the lateral direction (CD direction), and an oblique direction of the web, and the like. As used herein, the term "oblique direction" means a direction that forms an angle in a range of more than zero (0) degree to less than 90 degrees with the longitudinal direction or the lateral direction along the plane direction of the web. In consideration of the manufacturing convenience, the vibrating direction is preferably the lateral direction or the direction that forms an angle in a range of more than 0 degree and 45 degrees or less with respect to the lateral direction.

The amplitude of vibration of the nozzle or the bore member is substantially the same as the amplitude of the first entanglement portions in the obtained nonwoven fabric. Therefore, the amplitude of vibration of the nozzle or the bore member is determined in accordance with the amplitude of the first entanglement portion to be obtained. The length of meandering per cycle of the first entanglement portion is determined depending on the vibration speed of the nozzles or the bore member and the moving speed of the pattern formation support. Therefore, the vibration speed of the nozzle or the bore member is determined in accordance with the length of the meandering per cycle of the first entanglement portion to be obtained, also in consideration of the moving speed of the pattern formation support.

The vibration speed of the bore member can be increased up to approximately 100 m/min. Increasing the vibration speed of the bore member can increase a difference between the width X at the turn-around point of the entanglement portion, such as the first entanglement portion or the second entanglement portion, and the width Y of other parts of the entanglement portion.

The web after completing the second hydroentanglement treatment may be subjected to a drying process for removing moisture and used as the nonwoven fabric as it is. The dried web (nonwoven fabric) may be wound as needed. When being used as a sheet for an absorbent article, the nonwoven fabric is cut into predetermined dimensions.

When the web contains synthetic fibers and a part or the entire of fiber surface of the synthetic fiber is formed of a resin having a relatively low melting point, such as polyethylene, the drying process may also serve as a thermal bonding process for thermally bonding the fibers together with a low-melting point resin. The thermally bonding process is preferably performed at a temperature at which a resin with the lowest melting point among the thermoplastic resins constituting the surface of the synthetic fiber is melted or softened.

After completing the second hydroentanglement treatment, the dried web (nonwoven fabric) may be further subjected to a water-repellent treatment such that water-repellent agent is attached to the fibers. The water-repellent treatment may be performed, for example, when cotton from which a large amount of cotton wax is removed by the scouring and bleaching is used as the cellulose-based fibers. The water-repellent treatment may be performed such that, for example, 0.5 to 5 parts by mass of a commercially available water-repellent agent (for example, a fiber treatment agent for imparting the water repellency to the fiber) is attached to 100 parts by mass of the nonwoven fabric.

(Sheet for Absorbent Article)

The nonwoven fabric described above constitutes a sheet for an absorbent article. The nonwoven fabric may alone constitute the sheet for an absorbent article or may constitute the sheet for an absorbent article in combination with another nonwoven fabric, film, or the like. When the sheet for an absorbent article is a surface sheet, the nonwoven fabric may be used as the sheet for an absorbent article as it is. The sheet for an absorbent article may be provided in the form of integrating the nonwoven fabric and a liquid impermeable film.

(Applications of Sheet for Absorbent Article)

The sheet for an absorbent article of the present embodiment can be used as a member constituting an absorbent article. For example, the sheet for an absorbent article of the present embodiment can be used as a member that constitutes a surface sheet for an absorbent article (also referred to as a top sheet), an intermediate sheet, a sheet covering an absorbent core (also referred to as an SAP sheet or a core wrap sheet), a backsheet, or the like. As used herein, the term backsheet refers to a sheet which is provided facing a liquid impermeable sheet provided opposite to the surface sheet via an absorber, to thereby constitute an exposed surface on the opposite side to the surface sheet of the absorbent article. Therefore, the backsheet is the portion that may be in contact with a user's clothing. When the sheet for an absorbent article is used as the backsheet, the nonwoven fabric and the liquid impermeable sheet may be integrated together.

The sheet for an absorbent article of the present embodiment is preferably used as the surface sheet in order to make use of the unique tactile sensation of the cellulose-based fibers because the sheet for an absorbent article uses the nonwoven fabric that contains cellulose-based fibers in an amount of more than 90% by mass. Examples of absorbent articles include disposable diapers (including baby diapers and adult diapers), sanitary napkins, incontinence pads, and the like. The absorbent article is not limited to these, and thus the sheet for an absorbent article of the present embodiment can be used for any article used for the absorption of body fluids excreted from a human body.

(Absorbent Article)

An absorbent article will be described as another embodiment of the present disclosure. One example of the absorbent article of the present embodiment is an absorbent article that includes a surface sheet, a liquid impermeable sheet, and an absorber disposed between the surface sheet and the liquid impermeable sheet, wherein the surface sheet is the sheet for an absorbent article of the embodiment described above. Another example of the absorbent article of the present embodiment is an absorbent article that includes a surface sheet, a liquid impermeable sheet, an absorber disposed between the surface sheet and the liquid impermeable sheet, and a backsheet disposed to face a surface of the liquid impermeable sheet opposite to a surface thereof on the absorber side, wherein the surface sheet or the backsheet is the sheet for an absorbent article of the embodiment described above.

The surface sheet has a function of capturing an excreted body fluid and quickly moving the captured excreted body fluid to the absorber side. In the present embodiment, when the surface sheet is the sheet for an absorbent article of the embodiment described above, the surface sheet can make the user feel a good tactile sensation and it also exhibits good design effect when the user sees the sheet at the time of wearing the article.

The liquid impermeable sheet is, for example, a resin film. The liquid impermeable sheet may have air permeability or may not have air permeability.

The absorber may be, for example, an absorbent core covered with a core wrap sheet. The absorbent core is constituted of one or more members selected from a polymeric absorber (also referred to as SAP, which is generally powder), a pulverized pulp, a fiber aggregate, and a film. The core wrap sheet is selected from the nonwoven fabric and the film. Alternatively, the absorber may be composed only of an absorbent core without being covered by the core wrap sheet.

When the backsheet is provided, the backsheet may be the sheet for an absorbent article of the embodiment previously described. The backsheet may be integrated with the liquid impermeable sheet by an adhesive or the like. Since the backsheet is a portion that a user who grasps an absorbent article is apt to touch, if this backsheet is the sheet for an absorbent article of the present embodiment previously described, the sheet for the absorbent article gives good tactile sensation to the user who grasps this article, and makes him/her feel the aesthetic appearance. When the backsheet is the sheet for an absorbent article of the embodiment previously described, the amount of fluff attached to the clothing can be decreased.

The absorbent article of the present embodiment may include an intermediate sheet between the surface sheet and the absorber. The intermediate sheet is provided between the surface sheet and the absorber so that the excreted body fluid absorbed from the surface sheet is quickly moved to the absorber while being diffused and then absorbed in a larger area of the absorber. The intermediate sheet may be, for example, a nonwoven fabric that contains a hydrophilic fiber and a hydrophobic fiber.

As mentioned above, examples of absorbent articles include disposable diapers, sanitary napkins, incontinence pads, and the like. However, the absorbent article of the present embodiment is not limited thereto.

Examples

The present embodiment will be described below by way of Examples.

The following supports were prepared as the pattern formation supports.

- Plain weave support A (plain weave A): plain weave net composed of warp yarns (monofilaments) of 0.7 mm in yarn diameter, and weft yarns (monofilaments) of 0.7 mm in yarn diameter, at a weave density of 25/25 (yarns/inch)
- Plain weave support D (plain weave D): plain weave net composed of warp yarns (monofilaments) of 0.9 mm in yarn diameter, and weft yarns (monofilaments) of 1.0 mm in yarn diameter, at a weave density of 9/10 (yarns/inch), wherein every two warp yarns were arranged on the same condition while one weft yarn was shot over and under these two warp yarns.

(Example 1)

A parallel web having an intended basis weight of 35 g/m$^2$ was produced using only cotton (trade name: MSD, manufactured by MARUSAN INDUSTRY CO., LTD. ) having a fineness of 1.0 dtex to 5.0 dtex and a fiber length of 10 mm to 60 mm. The hydroentanglement treatment (the first hydroentanglement treatment) was performed on the entire web. In the first hydroentanglement treatment, the web was placed on a 90-mesh plain weave support composed of monofilaments each having a yarn diameter of 0.132mm. Then, by using a nozzle with orifices of 0.12 mm in hole diameter arranged at intervals of 0.6 mm, a columnar water stream was jetted at 2 MPa once onto one surface of the web, another columnar water stream was jetted at 4 MPa once onto the other surface thereof, and a further columnar water stream was jetted at 2 MPa once onto the one surface thereof. The speed of the support at this time was 4 m/minute, and the distance between the nozzle and the web was 10 mm.

Next, the hydroentanglement treatment (the second hydroentanglement treatment) for forming the first entanglement portion was performed. The second hydroentanglement treatment was performed by placing the web obtained after the first hydroentanglement treatment, on the plain weave support A. In the second hydroentanglement treatment, the same nozzle as that used in the first hydroentanglement treatment was also used. A 5-mm-thick bore member made of an acrylic resin was disposed between the nozzle and the nonwoven fabric, and the water stream was caused to pass through bores of the bore member, thereby forming the second entanglement portions. Specifically, the bore member made of the acrylic resin with the bores of 6 mm in width and 6 mm in length was used so that the first entanglement portions, each having a width of 6 mm, were formed at intervals of 12 mm (that is, the second entanglement portions, each having a width of 12 mm, and the first entanglement portions, each having a width of 6mm, were alternately formed in a stripe pattern).

The columnar water stream was jetted once onto one surface of the web by setting the pressure of the columnar water stream to 2 MPa. During the hydroentanglement treatment, the nozzle was not vibrated and the support was traveled at a speed of 4 m/min. The distance between the nozzle and the web was 30 mm, and the distance between the bore member and the web was 5 mm. The columnar water stream passed only through the bores of the bore member and hit the web on the support, so that the hydroentanglement was partially performed. In the plain weave support A, the highest parts at the woven crossing point formed convex portions that were substantially identical, and these convex portions were arranged in the staggered manner. Thus, the portions of the fibers located on the convex portions were moved toward the peripheries thereof by water stream, so that the low-density regions A were formed in the staggered manner, and at the same time the moved fibers were entangled together to form the high-density regions, thus forming the regular pattern.

The obtained nonwoven fabric had the first entanglement portions having the regular pattern and each of which had a width of 6 mm, and the second entanglement portions which were plain and each of which had a width of 12 mm. The regular pattern of the first entanglement portions included elliptical low-density regions A arranged in the staggered manner, each region having a major axis of 1.2 mm and a minor axis of 0.7 mm. Most of the low-density regions A were opening portions. The low-density regions A were arranged in the staggered manner with a pitch in the longitudinal direction of 3 mm, a pitch in the lateral direction of 2 mm, an interval in the longitudinal direction of 2 mm, an interval in the lateral direction of 1 mm, a pitch in the diagonal direction of 2 mm and an interval in the diagonal direction of 1 mm. The opening ratio of the entire nonwoven fabric was 9.20%.

(Example 2)

A nonwoven fabric was obtained by the same procedure as in Example 1 except that, in the second entanglement treatment, a bore member (of 5 mm in thickness) having a bore of 6 mm in width and 6 mm in length and made of an acrylic resin was used to form the first entanglement portions, each having a width of 6 mm, at intervals of 6 mm. The obtained nonwoven fabric had the first entanglement portions which had the regular pattern and each of which had a width of 6 mm, and the second entanglement portions which were plain and each of which had a width of 6mm. The regular pattern of the obtained nonwoven fabric was the same as that in Example 1, with the opening ratio of the entire nonwoven fabric being 13.90%.

(Example 3)

A nonwoven fabric was obtained by the same procedure as in Example 1 except that the intended basis weight was set to 40 g/m$^2$, and that a water stream was jetted onto one surface of a web at a water pressure of 2 MPa, another water stream was jetted onto the other surface thereof at a water pressure of 4 MPa, and a further water stream was jetted onto the one surface thereof at a water pressure of 2 MPa in the first hydroentanglement treatment. The regular pattern and porosity of the obtained nonwoven fabric were the same as those in Example 1.

(Example 4)

A nonwoven fabric was obtained by the same procedure as in Example 1 except that the intended basis weight was set to 40 g/m$^2$, a water stream was jetted onto one surface of a web at a water pressure of 2 MPa, another water stream was jetted onto the other surface thereof at a water pressure of 4 MPa, and a further water stream was jetted onto the one surface thereof at a water pressure of 2 MPa in the first hydroentanglement treatment, and also except that a bore member (of 5 mm in thickness) having a bore of 6 mm in width and 6 mm in length and made of an acrylic resin was used to form the first entanglement portions, each having a width of 6 mm, at intervals of 6 mm in the second hydroentanglement treatment. The regular pattern of the obtained nonwoven fabric was the same as that in Example 1, with the opening ratio of the entire nonwoven fabric being 13.90%.

(Example 5)

A nonwoven fabric was obtained by the same procedure as in Example 1 except that the intended basis weight was set to 45 g/m$^2$, and that a water stream was jetted onto one surface of a web at a water pressure of 2 MPa, another water stream was jetted onto the other surface thereof at a water pressure of 4 MPa, and a further water stream was jetted onto the one surface thereof at a water pressure of 2 MPa in the first hydroentanglement treatment. The regular pattern and opening ratio of the obtained nonwoven fabric were the same as those in Example 1.

(Example 6)

A nonwoven fabric was obtained by the same procedure as in Example 1 except that the intended basis weight was set to 45 g/m$^2$, a water stream was jetted onto one surface of a web at a water pressure of 2 MPa, another water stream was jetted onto the other surface thereof at a water pressure of 4 MPa, and a further water stream was jetted onto the one surface thereof at a water pressure of 2 MPa in the first hydroentanglement treatment, and also except that a bore member (of 5 mm in thickness) having a bore of 6 mm in width and 6 mm in length and made of an acrylic resin was used to arrange the first entanglement portions, each having a width of 6 mm, at intervals of 6 mm. The regular pattern of the obtained nonwoven fabric was the same as that in Example 1, with the opening ratio of the entire nonwoven fabric being 13.90%.

(Comparative Example 1)

A nonwoven fabric was obtained by the same procedure as in Example 4 except that the second entanglement treatment was not performed. The nonwoven fabric was plain across its entire surface and did not have any first entanglement portion.

(Comparative Example 2)

A nonwoven fabric was obtained by the same procedure as in Example 4 except that a water stream was jetted across the entire surface of a web without using the bore member in the second entanglement treatment. The nonwoven fabric had, over its entire surface, a regular pattern that had the low-density regions A arranged in the staggered manner and the high-density regions, and thus the nonwoven fabric did not have any second entanglement portion. The shape and dimensions of the low-density regions A arranged in the staggered manner and the interval and pitch between the low-density regions A were the same as those of the low-density regions A formed in the first entanglement portion of Example 1. The opening ratio of the entire nonwoven fabric was 28.00%.

(Comparative Example 3)

A nonwoven fabric was obtained by the same procedure as in Example 1 except that the intended basis weight was set to 40 g/m$^2$, and that a water stream was jetted onto one surface of a web at a water pressure of 2 MPa, another water stream was jetted onto the other surface thereof at a water pressure of 2 MPa, and a further water stream was jetted onto the one surface thereof at a water pressure of 2 MPa in the first hydroentanglement treatment and also except that the plain weave support D was used in the second hydroentanglement treatment. The opening ratio of the entire nonwoven fabric was 5.97%.

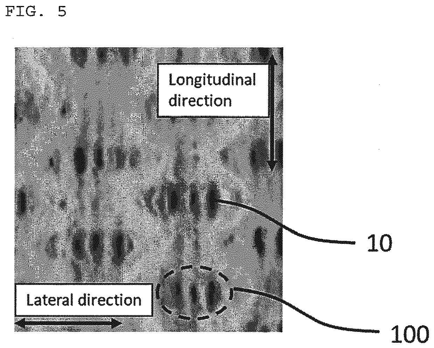

The nonwoven fabric obtained in Comparative Example 3 had a regular pattern formed in the first entanglement portion as shown in FIG. 5. As shown in FIG. 5, three low-density regions 10 that were substantially identical were arranged side by side in the lateral direction of the nonwoven fabric to constitute one group. The groups 100 were arranged in the staggered manner. Therefore, in Comparative Example 3, one low-density region and all other low-density regions that are adjacent to and substantially identical to the one low-density region did not have a relationship of either a staggered arrangement or a lattice arrangement therebetween.

The following properties of the nonwoven fabrics of Examples 1 to 6 and Comparative Examples 1 to 3 were evaluated by methods described below. The evaluation results are shown in Table 1.

(Thickness, Specific Volume)

The thickness of a sample of the nonwoven fabric was measured using a pachymeter (trade name: THICKNESS GAUGE Model CR-60A, manufactured by DAIEI KAGAKU SEIKI MFG. CO., LTD.) under a load of 2.94 cN per cm$^2$ of the sample. The specific volume of the sample was determined by calculation from the basis weight and the thickness thereof.

(Run-Off Value)

The run-off value of the sample was measured in accordance with the following procedure.

(i) A sample is prepared.
(ii) A slope that forms an angle of 30 degrees with respect to a horizontal plane is prepared. Two sheets of filter paper (Lister Paper (Grade 989, each 10 cm × 10 cm) are stacked on the slope and prepared as an absorber. Specifically, the absorbers are laid over an area of the slope that is equal to or greater than the area of the sample so that they do not overlap each other. The sample is placed and fixed onto the absorbers. The sample is disposed on the slope such that the extending directions of the first entanglement portion and the second entanglement portion are orthogonal to an inclination direction of the slope.
(iii) Then, 1.0 g or 3.0 g of 0.90% saline (colored with blue dyes) at 37°C was dropped at a position of 1cm below an upper end of the sample from a microtube pump or burette at a rate of 1.0 g/30 sec. When the whole saline is absorbed in the nonwoven fabric sample and droplets of the saline disappear from the surface of the nonwoven fabric sample, the position of the tip of the saline is measured. The run-off value is obtained by determining a distance between the above-mentioned position and a position on a surface of the nonwoven fabric sample at which the saline has been dropped, i.e., the longest distance by which the droplets of the saline had flowed on the surface of the nonwoven fabric sample.

Three samples were prepared for one nonwoven fabric. The values obtained by repeating a set of the processes (i) to (iii) three times were averaged, and the average value was used for evaluation as a run-off value of the nonwoven fabric.

[Table 1]

| | Width of first entanglement portion/width of second entanglement portion | Intended basis weight (g/m²) | Basis weight (g/m²) | Thickness (mm) | Run-off value (mm)/dropped amount of 1 g | | | | Run-off value (mm)/dropped amount of 3 g | | | |
|---|---|---|---|---|---|---|---|---|---|---|---|---|
| | | | | | Sample 1 | Sample 2 | Sample 3 | Average | Sample 1 | Sample 2 | Sample 3 | Average |
| Example 1 | 6 mm/12 mm | 35 | 34.7 | 0.38 | 43 | 39 | 43 | 41.7 | - | - | - | - |
| Example 2 | 6 mm/6 mm | 35 | 36.3 | 0.38 | 43 | 45 | 44 | 44.0 | 68 | 66 | 78 | 70.7 |
| Example 3 | 6 mm/12 mm | 40 | 40.0 | 0.42 | 38 | 41 | 33 | 37.3 | 68 | 68 | 71 | 69.0 |
| Example 4 | 6 mm/6 mm | 40 | 39.5 | 0.42 | 35 | 41 | 40 | 38.7 | 72 | 67 | 72 | 70.3 |
| Example 5 | 6 mm/12 mm | 45 | 45.1 | 0.43 | 40 | 37 | 35 | 37.3 | - | - | - | - |
| Example 6 | 6 mm/6 mm | 45 | 44.3 | 0.43 | 35 | 34 | 40 | 36.3 | - | - | - | - |
| Comparative Example 1 | Entire plain surface | 40 | 39.1 | 0.39 | - | - | - | - | 71 | 73 | 77 | 73.7 |
| Comparative Example 2 | Entire regular pattern | 40 | 42.3 | 0.45 | - | - | - | - | 73 | 71 | 71 | 71.7 |
| Comparative Example 3 | 6 mm/6 mm | 40 | 40.1 | 0.43 | - | - | - | - | 73 | 78 | 83 | 78.0 |

EP 3 646 835 B1

According to the comparison among Examples 1, 3 and 5, and the comparison among Examples 2, 4 and 6, in a case where the first entanglement portions had the same width, the nonwoven fabric having the larger basis weight tended to exhibit a smaller run-off value, that is, as the basis weight became larger, the liquid absorption rate tended to be improved. Regarding the run-off value when setting the liquid dropped amount to 3 g, both Examples 3 and 4, each of which had the first entanglement portions and the second entanglement portions, exhibited a run-off value smaller than a run-off value of Comparative Example 1 in which the entire surface of the nonwoven fabric was plain and of Comparative Example 2 in which the nonwoven fabric had the regular pattern over its surface. In addition, Examples 3 and 4 also exhibited a run-off value smaller than a run-off value of Comparative Example 3, which showed that the water absorbability was affected depending on the arrangement manner of the low-density regions A.

(Example 7)

A parallel web having an intended basis weight of 35 $g/m^2$ was produced using only cotton (trade name: MSD, manufactured by MARUSAN INDUSTRY CO., LTD.) having a fineness of 1.0 dtex to 5.0 dtex and a fiber length of 10 mm to 60 mm. The hydroentanglement treatment (the first hydroentanglement treatment) was performed on the entire web. The first hydroentanglement treatment was performed by placing the web on a 90-mesh plain weave support composed of monofilaments having a yarn diameter of 0.132 mm so that the total energy (E) applied to the web by the high pressure water was 74.7 Wh/kg/m. The distance between the nozzle and the web was 30 mm.

Next, the hydroentanglement treatment (the second hydroentanglement treatment) for forming the first entanglement portions was performed. The second hydroentanglement treatment was performed by placing the web obtained after the first hydroentanglement treatment, on the plain weave support A. In the second hydroentanglement treatment, the same nozzle as that used in the first hydroentanglement treatment was also used. A 5-mm-thick bore member made of an acrylic resin was disposed between the nozzle and the nonwoven fabric, and the water stream was caused to pass through the bores of the bore member, thereby forming the second entanglement portions. Specifically, the bore member made of an acrylic resin with the bores of 6 mm in width and 6 mm in length was used so that the first entanglement portions, each having a width of 6 mm, were formed at intervals of 12 mm (that is, the second entanglement portions, each having a width of 12 mm, and the first entanglement portions, each having a width of 6 mm, were alternately formed in a stripe pattern).

The second hydroentanglement treatment was performed such that the total energy (E) applied to the web by the high pressure water was 32.8 Wh/kg/m. During the hydroentanglement treatment, the bore member was vibrated at an amplitude of 30 mm and a vibration speed of 10.5 m/min along the lateral direction of the web. The distance between the nozzle and the web was 5 mm. Also in this example, the hydroentanglement treatment was partially performed in the same way as in Example 1. Thus, like Example 1, in the first entanglement portion, the low-density regions A were formed to be arranged in the staggered manner, and at the same time, the moved fibers were entangled each other to form the high-density regions, thereby forming the regular pattern. Further, the vibration of the nozzle made the first entanglement portions and the second entanglement portions meandering.

The obtained nonwoven fabric had the first entanglement portions which had the regular pattern and each of which had a width of 6 mm, and the second entanglement portions which were plain and each of which had a width of 12 mm. The regular pattern in the first entanglement portion included elliptical low-density regions A arranged in the staggered manner, each region having a major axis of 1.2 mm and a minor axis of 0.7 mm. Most of the low-density regions A were opening portions. The low-density regions A were arranged in the staggered manner with a pitch in the longitudinal direction of 3 mm, a pitch in the lateral direction of 2 mm, an interval in the longitudinal direction of 2 mm, an interval in the lateral direction of 1 mm, a pitch in the diagonal direction of 2 mm and an interval in the diagonal direction of 1 mm. The length of the meandering per cycle and amplitude of the first entanglement portion was 105 mm and 15 mm, respectively. The opening ratio of the entire nonwoven fabric was 9.20%.

(Example 8)

A nonwoven fabric was obtained by the same procedure as in Example 7 except that the basis weight was set to 40 $g/m^2$ and the first hydroentanglement treatment was performed such that the total energy (E) applied to the web by high pressure water was 39.6 Wh/kg/m.

(Example 9)

A nonwoven fabric was obtained by the same procedure as in Example 7 except that the first hydroentanglement treatment was performed such that the total energy (E) applied to the web by the high pressure water was 52.8 Wh/kg/m.

(Comparative Example 4)

A nonwoven fabric was obtained by the same procedure as in Example 1 except that the intended basis weight was set to 30 g/m$^2$ and the first hydroentanglement treatment was performed such that the total energy (E) applied to the web by high pressure water was 21.9 Wh/kg/m. When the intended basis weight was set to 30 g/m$^2$ and the conditions for the first hydroentanglement treatment were set to be the same as those used in Example 7, the texture of the nonwoven fabric was disturbed due to a large energy of the high pressure water, significantly deteriorating an external appearance of the nonwoven fabric.

(Comparative Example 5)

A nonwoven fabric was obtained by the same procedure as in Example 1 except that the basis weight was set to 30g/m$^2$ and the first hydroentanglement treatment was performed such that the total energy (E) applied to the web by high pressure water was 18.8 Wh/kg/m.

The nonwoven fabrics of Examples 7 to 9 and Comparative Examples 4 and 5 were evaluated for the thickness, specific volume, fluff falling amount, and breaking strength. The evaluation results are shown in Table 2. An evaluation method of the thickness of the nonwoven fabric has been explained above. The fluff amount and the breaking strength of the nonwoven fabric were evaluated by methods described below. The evaluation results are shown in Table 2. In Table 2, the evaluation results of Comparative Examples 2 and 3 are also shown.

(Fluff Falling Amount Measurement Test)

a) A disk (of 70 mm in diameter and 350 g in weight) having its surface covered with urethane foam (manufactured by Bridgestone Corporation, trade name: MOLTOPREN MF30, 5 mm in thickness) is attached to a rotating shaft so that the rotating shaft is positioned 20 mm away from the center of the disk.
b) An urethane foam that is of the same kind as the above-mentioned urethane foam is laid on a lower surface of a nonwoven fabric, and then the nonwoven fabric is fixed on a stand with an upper surface of the nonwoven fabric being an exposed surface.
c) The disk is placed on the nonwoven fabric. At this time, a load applied to the nonwoven fabric is only a self-weight of the disk.
d) The disk is revolved on the nonwoven fabric by rotating the rotating shaft. Four sets of the revolutions are performed such that each set included two clockwise rotations and two counterclockwise rotations. The revolution rate at this time is approximately three seconds per revolution.
e) After the four sets of revolutions, the fibers falling off the nonwoven fabric and attached to the surface of the urethane foam covering the disk are collected.
f) The processes a) to e) mentioned above were performed on 30 pieces of the nonwoven fabrics, i.e., n = 30. The mass of the fibers falling off each of 30 pieces of nonwoven fabrics was measured, and finally an average of the measured masses was referred to as a fluff falling amount.

(Breaking Strength)

A load value (breaking strength) of a sample piece of the nonwoven fabric at the time of breakage was measured by a tensile test under the conditions that the width of the sample piece was 5 cm, a gripping interval was 10 cm, and the tensile speed was 30 $\pm$ 2 cm/min using a constant-rate-of-traverse type tensile tester in accordance with JIS L 1096 6.12.1 A (strip method). The tensile test was performed by setting the longitudinal direction (MD direction) and the lateral direction (CD direction) of the nonwoven fabric as tensile directions. Each of the evaluation results indicates an average of the values measured for three samples.

[Table 2]

| | Width of first entanglement portion/width of second entanglement portion | Intended basis weight (g/m²) | Basis weight (g/m²) | Fluff amount (mg) | Thickness (mm) | Breaking strength | |
|---|---|---|---|---|---|---|---|
| | | | | | | Longitudinal direction (N/ 5 cm) | Lateral direction (N/5 cm) |
| Example 7 | 6 mm/12 mm | 35 | 34.8 | 0.1000 | 0.36 | 30.1 | 10.6 |
| Example 8 | 6 mm/12 mm | 40 | 40.8 | 0.1000 | 0.41 | 31.7 | 11.6 |
| Example 9 | 6 mm/12 mm | 35 | 33.3 | 0.1500 | 0.38 | 23.6 | 8.5 |
| Comparative Example 4 | 6 mm/12 mm | 30 | 29.5 | 1.5000 | 0.37 | 20.8 | 7.6 |
| Comparative Example 5 | 6 mm/12 mm | 30 | 29.1 | 1.2000 | 0.34 | 24.8 | 9.6 |
| Comparative Example 2 | Entire plain surface | 40 | 40.2 | 2.8000 | 0.45 | 29.8 | 7.5 |
| Comparative Example 3 | Entire regular pattern | 40 | 39.8 | 0.1000 | 0.39 | 32.8 | 13.0 |

Each of the nonwoven fabrics of Examples 7 to 9 that had a basis weight of more than 30 g/m² had a small fluff falling amount. In Examples 7 to 9, the texture of the second entanglement portion was good. The nonwoven fabrics of Comparative Examples 4 and 5 in which the basis weight was 30 g/m² or less had a weak entanglement of the fibers because the total energy (E) applied to the web by the high pressure water was made lower in the first hydroentanglement treatment to ensure the good texture of the nonwoven fabric. For this reason, these nonwoven fabrics had a large fluff falling amount even though the first entanglement portions having the regular pattern were formed. When comparing the nonwoven fabrics of Examples 7 to 9 with the nonwoven fabrics of Comparative Examples 4 and 5, the nonwoven fabrics of Comparative Examples 4 and 5 were observed to have more fluffing.

The nonwoven fabric of Comparative Example 2 did not have any first entanglement portion and thus exhibited the largest fluff falling amount. The nonwoven fabric of Comparative Example 3 had the regular pattern formed across its entire surface and thus exhibited a small fluff falling amount. However, since the high-density region was formed across its entire nonwoven fabric, this nonwoven fabric exhibited a large amount of liquid return, compared to the nonwoven fabrics of Examples. Because the fluff falling amount in Comparative Example 3 was substantially the same as or slightly smaller than that of each of Examples 7 to 9, it was confirmed that the presence of the second entanglement portions did not significantly reduce the fluff falling.

Industrial Applicability

The sheet for an absorbent article of the present embodiment has high-level design effect while suppressing the fluffing and fluff falling, even though it contains cellulose-based fibers in an amount of more than 90% by mass. Therefore, the sheet for an absorbent article of the present embodiment is suitably used as, for example, a surface sheet or a backsheet in the absorbent article that has the surface sheet, a backsheet, and an absorber disposed between the surface sheet and the backsheet.

The present disclosure includes a sheet for an absorbent article and an absorbent article according to the following aspects.

(First Aspect)

A sheet for an absorbent article, including a nonwoven fabric containing cellulose-based fibers in an amount of more than 90% by mass, the nonwoven fabric being formed by entangling fibers,

wherein the nonwoven fabric includes a plurality of first entanglement portions spaced apart from each other and a plurality of second entanglement portions spaced apart from each other,
each of the first entanglement portions has a regular pattern formed by a plurality of regions having a high fiber density (hereinafter referred to as "high-density regions") and a plurality of regions having a low fiber density (hereinafter referred to as "low-density regions"), the regular pattern having substantially identical low-density regions arranged in a staggered or lattice manner,

each of the first entanglement portions extends along a longitudinal direction or a lateral direction of the nonwoven fabric,

each of the second entanglement portions extends along the longitudinal direction or the lateral direction of the nonwoven fabric,

the first entanglement portions and the second entanglement portions are alternately arranged,

a combined area of the first entanglement portions is 20% or more of the entire area of the nonwoven fabric,

a basis weight of the nonwoven fabric is more than 30 g/m$^2$, and

a fluff falling amount in the following test is 1.0 mg or less.

(Fluff Falling Amount Measurement Test)

a) A disk (of 70 mm in diameter and 350 g in weight) having its surface covered with urethane foam (manufactured by Bridgestone Corporation, trade name: MOLTOPREN MF30, 5 mm in thickness) is attached to a rotating shaft so that the rotating shaft is positioned 20 mm away from the center of the disk.

b) An urethane foam that is of the same kind as the above-mentioned urethane foam is laid on a lower surface of the nonwoven fabric, and then the nonwoven fabric is fixed on a stand with an upper surface of the nonwoven fabric being an exposed surface.

c) The disk is placed on the nonwoven fabric. At this time, a load applied to the nonwoven fabric is only a self-weight of the disk.

d) The disk is revolved on the nonwoven fabric by rotating the rotating shaft. Four sets of the revolutions are performed such that each set includes two clockwise rotations and two counterclockwise rotations. The revolution rate at this time is approximately three seconds per revolution.

e) After the four sets of revolutions, the fibers falling off the nonwoven fabric and attached to the surface of the urethane foam covering the disk are collected.

f) The processes a) to e) mentioned above are performed on 30 pieces of the nonwoven fabrics, i.e., n = 30. The mass of the fibers falling off each of the 30 pieces of nonwoven fabrics is measured, and finally an average of the measured masses is referred to as a fluff falling amount.

(Second Aspect)

The sheet for an absorbent article according to the first aspect, wherein the fibers are not bonded together.

(Third Aspect)

The sheet for an absorbent article according to the first or second aspect, wherein the cellulose-based fiber is cotton.

(Fourth Aspect)

The sheet for an absorbent article according to the first or second aspect, wherein the cellulose-based fiber is cotton having water repellency.

(Fifth Aspect)

The sheet for an absorbent article according to any one of the first to fourth aspects, including only the cellulose-based fibers.

(Sixth Aspect)

The sheet for an absorbent article according to any one of the first to fifth aspects, wherein the substantially identical low-density regions are opening portions.

(Seventh Aspect)

The sheet for an absorbent article according to any one of the first to sixth aspects, wherein one low-density region has an area of 0.1 mm$^2$ to 10 mm$^2$.

(Eighth Aspect)

The sheet for an absorbent article according to any one of the first to seventh aspects, wherein a width of the first entanglement portion is in a range of 5 mm to 50 mm, while a width of the second entanglement portion is in a range of 5 mm to 50 mm.

(Ninth Aspect)

The sheet for an absorbent article according to any one of the first to eighth aspects, wherein the sheet for an absorbent article is a surface sheet or a backsheet.

(Tenth Aspect)

An absorbent article including a surface sheet, a liquid impermeable sheet, and an absorber disposed between the surface sheet and the liquid impermeable sheet, wherein the surface sheet is the sheet for an absorbent article according to any one of the first to eighth aspects.

(Eleventh Aspect)

An absorbent article including a surface sheet, a liquid impermeable sheet, an absorber disposed between the surface sheet and the liquid impermeable sheet, and a backsheet disposed to face a surface of the liquid impermeable sheet, wherein the surface of the liquid impermeable sheet is opposite to an absorber-side surface thereof, and the surface sheet or the backsheet is the sheet for an absorbent article according to any one of the first to eighth aspects.

Description of Reference Symbols

    10   Low-density region A
    20   Low-density region A
    31   Direction in which a first entanglement portion is meandering
    32   Direction orthogonal to the direction in which the first entanglement portion is meandering
    41   First entanglement portion
    42   Second entanglement portion


**Claims**

1. A sheet for an absorbent article, comprising a nonwoven fabric containing cellulose-based fibers in an amount of more than 90% by mass, the nonwoven fabric being formed by entangling fibers,

   wherein the nonwoven fabric includes a plurality of first entanglement portions (42) spaced apart from each other and a plurality of second entanglement portions (41) spaced apart from each other,
   each of the first entanglement portions (42) has a regular pattern formed by a plurality of regions having a high fiber density, hereinafter referred to as "high-density regions", and a plurality of regions (10, 20) having a low fiber density, hereinafter referred to as "low-density regions", the regular pattern having substantially identical low-density regions (10, 20) arranged in a staggered or lattice manner,
   each of the first entanglement portions (42) extends along a longitudinal direction (31) or a lateral direction (32a, 32b) of the nonwoven fabric,
   each of the second entanglement portions (41) extends along the longitudinal direction (31) or the lateral direction (32a, 32b) of the nonwoven fabric,
   the first entanglement portions (42) and the second entanglement portions (41) are alternately arranged,
   a combined area of the first entanglement portions (42) is 20% or more of the entire area of the nonwoven fabric,

a basis weight of the nonwoven fabric is more than 30 g/m$^2$, and
a fluff falling amount in the Fluff Falling Amount Measurement Test, as described in the description, is 1.0 mg or less.

2. The sheet for an absorbent article according to claim 1, wherein the fibers are not bonded together.

3. The sheet for an absorbent article according to claim 1 or 2, wherein the cellulose-based fiber is cotton.

4. The sheet for an absorbent article according to claim 1 or 2, wherein the cellulose-based fiber is cotton having water repellency.

5. The sheet for an absorbent article according to any one of claims 1 to 4, comprising only the cellulose-based fibers.

6. The sheet for an absorbent article according to any one of claims 1 to 5, wherein the substantially identical low-density regions (10, 20) are opening portions.

7. The sheet for an absorbent article according to any one of claims 1 to 6, wherein one low-density region (10, 20) has an area of 0.1 mm$^2$ to 10 mm$^2$.

8. The sheet for an absorbent article according to any one of claims 1 to 7, wherein a width of the first entanglement portion (42) is in a range of 5 mm to 50 mm, while a width of the second entanglement portion (41) is in a range from 5 mm to 50 mm.

9. The sheet for an absorbent article according to any one of claims 1 to 8, wherein the sheet for an absorbent article is a surface sheet or a backsheet.

10. An absorbent article comprising a surface sheet, a liquid impermeable sheet, and an absorber disposed between the surface sheet and the liquid impermeable sheet, wherein the surface sheet is the sheet for an absorbent article according to any one of claims 1 to 8.

11. An absorbent article comprising a surface sheet, a liquid impermeable sheet, an absorber disposed between the surface sheet and the liquid impermeable sheet, and a backsheet disposed to face a surface of the liquid impermeable sheet, wherein the surface of the liquid impermeable sheet is opposite to an absorber-side surface thereof, and the surface sheet or the backsheet is the sheet for an absorbent article according to any one of claims 1 to 8.

**Patentansprüche**

1. Blatt für einen saugfähigen Artikel, mit einem Vliesgewebe mit Zellulose-basierten Fasern in einer Menge von mehr als 90 Massenprozent, wobei das Vliesgewebe durch Verschlingen von Fasern gebildet ist,

wobei das Vliesgewebe mehrere erste Verschlingungsabschnitte (42), die voneinander beabstandet sind, und mehrere zweite Verschlingungsabschnitte (41) aufweist, die voneinander beabstandet sind,
jeder der ersten Verschlingungsabschnitte (42) ein regelmäßiges Muster aufweist, das durch mehrere Regionen mit einer hohen Faserdichte, die im Folgenden als "hochdichte Bereiche" bezeichnet werden, und mehrere Bereiche (10, 20) mit einer niedrigen Faserdichte gebildet ist, die im Folgenden als "niederdichte Bereiche" bezeichnet werden, wobei das regelmäßige Muster im wesentlichen identische niederdichte Bereiche (10, 20) aufweist, die in einer gestaffelten oder gitterartigen Weise angeordnet sind,
sich jeder der ersten Verschlingungsabschnitte (42) entlang einer Längsrichtung (31) oder einer Querrichtung (32a, 32b) des Vliesgewebes erstreckt,
sich jeder der zweiten Verschlingungsabschnitte (41) sich entlang der Längsrichtung (31) oder der Querrichtung (32, 32b) des Vliesgewebes erstreckt,
die ersten Verschlingungsabschnitte (42) und die zweiten Verschlingungsabschnitte (41) abwechselnd angeordnet sind,
eine kombinierte Fläche der ersten Verschlingungsabschnitte (42) 20% oder mehr der Gesamtfläche des Vliesgewebes ausmacht,
ein Basisgewicht des Vliesgewebes mehr als 30 g/m$^2$ beträgt, und
eine Flusenfallmenge in dem Flusenfallmengenmesstest, wie er in der Beschreibung beschrieben ist, 1,0 mg oder weniger beträgt.

2. Blatt für einen saugfähigen Artikel nach Anspruch 1, wobei die Fasern nicht zusammen gebondet sind.

3. Blatt für einen saugfähigen Artikel nach Anspruch 1 oder 2, wobei die Zellulose-basierte Faser Baumwolle ist.

4. Blatt für einen saugfähigen Artikel nach Anspruch 1 oder 2, wobei die Zellulose-basierte Faser Baumwolle mit einem Wasserabweisungsvermögen ist.

5. Blatt für einen saugfähigen Artikel nach einem der Ansprüche 1 bis 4, umfassend lediglich die Zellulose-basierten Fasern.

6. Blatt für einen saugfähigen Artikel nach einem der Ansprüche 1 bis 5, wobei die im Wesentlichen identischen niederdichte Bereiche (10, 20) Öffnungsabschnitte sind.

7. Blatt für einen saugfähigen Artikel nach einem der Ansprüche 1 bis 6, wobei ein niederdichter Bereich (10, 20) eine Fläche von 0,1 mm$^2$ bis 10 mm$^2$ aufweist.

8. Blatt für einen saugfähigen Artikel nach einem der Ansprüche 1 bis 7, wobei eine Breite des ersten Verschlingungsabschnitts (42) in einem Bereich von 5 mm bis 50 mm liegt, während eine Breite des zweiten Verschlingungsabschnitts (41) in einem Bereich von 5 mm bis 50 mm liegt.

9. Blatt für einen saugfähigen Artikel nach einem der Ansprüche 1 bis 8, wobei das Blatt für einen saugfähigen Artikel ein Oberflächenblatt oder ein Rückseitenblatt ist.

10. Saugfähiger Artikel mit einem Oberflächenblatt, einem für Flüssigkeit undurchlässigem Blatt und einem Absorber, der zwischen dem Oberflächenblatt und dem für Flüssigkeit undurchlässigen Blatt angeordnet ist, wobei das Oberflächenblatt das Blatt für einen saugfähigen Artikel nach einem der Ansprüche 1 bis 8 ist.

11. Saugfähiger Artikel mit einem Oberflächenblatt, einem für Flüssigkeit undurchlässigen Blatt, einem Absorber, der zwischen dem Oberflächenblatt und dem für Flüssigkeit undurchlässigen Blatt angeordnet ist, und einem Rückseitenblatt, das angeordnet ist, einer Oberfläche des für Flüssigkeit undurchlässigen Blatts gegenüberzuliegen, wobei die Oberfläche des für Flüssigkeit undurchlässigen Blatts einer Absorber-seitigen Oberfläche davon gegenüberliegt, und das Oberflächenblatt oder das Rückseitenblatt das Blatt für einen saugfähigen Artikel nach einem der Ansprüche 1 bis 8 ist.

## Revendications

1. Feuille pour un article absorbant, comprenant un tissu non tissé contenant des fibres à base de cellulose en une quantité de plus de 90 % en masse, le tissu non tissé étant formé par l'enchevêtrement de fibres,

dans laquelle le tissu non tissé comporte une pluralité de premières parties d'enchevêtrement (42) espacées les unes des autres et une pluralité de deuxièmes parties d'enchevêtrement (41) espacées les unes des autres, chacune des premières parties d'enchevêtrement (42) a un motif régulier formé d'une pluralité de régions ayant une haute densité de fibres, ci-après dénommées "régions à haute densité", et une pluralité de régions (10, 20) ayant une faible densité de fibres, ci-après dénommées "régions à faible densité", le motif régulier ayant des régions à faible densité (10, 20) sensiblement identiques agencées en quinconce ou en treillis, chacune des premières parties d'enchevêtrement (42) s'étend le long d'une direction longitudinale (31) ou d'une direction latérale (32a, 32b) du tissu non tissé, chacune des deuxièmes parties d'enchevêtrement (41) s'étend le long de la direction longitudinale (31) ou de la de la direction latérale (32a, 32b) du tissu non tissé, les premières parties d'enchevêtrement (42) et les deuxièmes parties d'enchevêtrement (41) sont agencées en alternance, une superficie combinée des premières parties d'enchevêtrement (42) fait 20 % ou plus la superficie totale du tissu non tissé, un poids de base du tissu non tissé est de plus de 30 g/m$^2$, et une quantité de peluche tombant lors du test de mesure de quantité de peluche tombant, tel que décrit dans la description, est de 1,0 mg ou moins.

2. Feuille pour un article absorbant selon la revendication 1, dans laquelle les fibres ne sont pas liées entre elles.

3. Feuille pour un article absorbant selon la revendication 1 ou 2, dans laquelle la fibre à base de cellulose est du coton.

4. Feuille pour un article absorbant selon la revendication 1 ou 2, dans laquelle la fibre à base de cellulose est du coton hydrophobe.

5. Feuille pour un article absorbant selon l'une quelconque des revendications 1 à 4, comprenant uniquement la fibre à base de celluloses.

6. Feuille pour un article absorbant selon l'une quelconque des revendications 1 à 5, dans laquelle les régions à faible densité (10, 20) sensiblement identiques sont des parties d'ouverture.

7. Feuille pour un article absorbant selon l'une quelconque des revendications 1 à 6, dans laquelle une région à faible densité (10, 20) a une superficie de 0,1 mm$^2$ à 10 mm$^2$.

8. Feuille pour un article absorbant selon l'une quelconque des revendications 1 à 7, dans laquelle une largeur de la première partie d'enchevêtrement (42) est dans une plage de 5 mm à 50 mm, tandis qu'une largeur de la deuxième partie d'enchevêtrement (41) est dans une plage de 5 mm à 50 mm.

9. Feuille pour un article absorbant selon l'une quelconque des revendications 1 à 8, dans laquelle la feuille pour un article absorbant est une feuille de surface ou une feuille inférieure.

10. Article absorbant comprenant une feuille de surface, une feuille imperméable aux liquides, et un absorbeur disposé entre la feuille de surface et la feuille imperméable aux liquides, dans lequel la feuille de surface est la feuille pour un article absorbant selon l'une quelconque des revendications 1 à 8.

11. Article absorbant comprenant une feuille de surface, une feuille imperméable aux liquides, un absorbeur disposé entre la feuille de surface et la feuille imperméable aux liquides, et une feuille inférieure disposée face à une surface de la feuille imperméable aux liquides, dans lequel la surface de la feuille imperméable aux liquides est opposée à une surface côté absorbeur de celle-ci, et la feuille de surface ou la feuille inférieure est la feuille pour un article absorbant selon l'une quelconque des revendications 1 à 8.

FIG. 1

FIG. 2

FIG. 3

FIG. 4

Second entanglement portion

First entanglement portion

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016112155 A
- JP 4893256 B